(19) **Europäisches Patentamt / European Patent Office / Office européen des brevets**

(11) **EP 1 749 891 A1**

(12) # EUROPEAN PATENT APPLICATION
published in accordance with Art. 158(3) EPC

(43) Date of publication:
**07.02.2007 Bulletin 2007/06**

(51) Int Cl.:
*C12Q 1/68* (2006.01)　　*C12N 9/50* (2006.01)
*A61K 38/43* (2006.01)

(21) Application number: **05774493.0**

(22) Date of filing: **27.05.2005**

(86) International application number:
**PCT/ES2005/070073**

(87) International publication number:
**WO 2005/116247 (08.12.2005 Gazette 2005/49)**

(84) Designated Contracting States:
**AT BE BG CH CY CZ DE DK EE ES FI FR GB GR HU IE IS IT LI LT LU MC NL PL PT RO SE SI SK TR**

(30) Priority: **27.05.2004 ES 200401281**
**15.11.2004 ES 200402737**

(71) Applicant: **Neocodex**
**41020 Sevilla (ES)**

(72) Inventors:
  • **RAMÍREZ-LORCA, Reposo**
  **C/ Averroes no. 8**
  **E-41020 Sevilla (ES)**
  • **GALÁN, Jose Jorge**
  **C/ Averroes no. 8**
  **E-41020 Sevilla (ES)**

  • **SÁEZ, Maria Eugenia**
  **C/ Averroes no. 8.**
  **E-41020 Sevilla (ES)**
  • **REAL, Luis Miguel**
  **C/ Averroes no. 8**
  **E-41020 Sevilla (ES)**
  • **RUIZ, Agustín**
  **C/ Averroes no. 8**
  **E-41020 Sevilla (ES)**

(74) Representative: **Elzaburu Marquez, Alberto**
**Elzaburu S.A.,**
**Miguel Angel, 21**
**28010 Madrid (ES)**

(54) **METHOD AND DEVICE FOR THE IN VITRO DETECTION OF POLYCYSTIC OVARIAN SYNDROME (PCOS) AND PATHOLOGIES INVOLVING CARDIOVASCULAR RISK**

(57)　The invention relates to a method and device for the in vitro detection of polycystic ovary syndrome (PCOS) and pathologies involving cardiovascular risk. An *in vitro* assay method and assay device (kit) for the diagnosis of the presence or the predisposition to suffer from PCOS and/or cardiovascular risk factors including: general obesity, abdominal obesity, hypertension, glucose intolerance, diabetes, hyperinsulinemia, general hypercholesterolemia, hypercholesterolemia with high LDL-cholesterol levels, low HDL-cholesterol levels and hypertriglyceridemia, as well as the grouping of some of these cardiovascular risk factors known as metabolic syndrome. The method and the kit are **characterized in that** they are based on the detection of at least one genotype or haplotype of a polymorphism in the *CAPN5* gene selected from: Nt g.86 A>G, Nt g.344 G>A, Nt c.1320 C>T and Nt c.1469 G>A or combinations thereof.

**Description**

**Field of the Invention**

The object of the present invention is the *in vitro* diagnosis of the so-called polycystic ovary syndrome (PCOS) or the diagnosis of the predisposition to develop this syndrome, as well as the detection of the existence of or the predisposition to develop certain pathologies representing cardiovascular risk factors and which frequently occur associated to the polycystic ovary syndrome. The *in vitro* diagnosis method is based on the detection of CAPN5 (calpain-5) gene polymorphisms. The presence or predisposition to develop PCOS itself, hypercholesterolemia (both linked to total cholesterol and specifically referring to LDL-cholesterol), high blood pressure (specifically a rise in diastolic pressure), obesity, glucose intolerance, diabetes, hypertriglyceridemia, and low HDL-cholesterol levels, as well as the grouping of some of these cardiovascular risk factors, known as metabolic syndrome, can be diagnosed by means of the assay method and the assay device of the invention.

**State of the Art**

The polycystic ovary syndrome (PCOS) is the most usual endocrine disorder that affects women of child-bearing age. A prospective study reported an overall presence of 6.5% of PCOS specifically in Spain.

Despite this prevalence, little is known about the etiology of PCOS but there is increasing evidence of an important genetic implication (Legro *et al.,* 1998; Govind *et al.,* 1999). However, the hereditary manner of PCOS is still uncertain and recent studies indicate that this disorder could be a complex character (Crosignani and Nicolosi, 2001). This means that several genes are interacting with environmental factors (especially dietary factors) to determine the typically heterogeneous, clinical and biochemical phenotype (Weiss and Terwilliger, 2000). Biochemical parameters including fasting insulin levels or hyperandrogenemia appear to be highly hereditary parameters, suggesting that some clinical signs, symptoms or biochemical parameters of PCOS could be transmitted as autosomal dominant or X chromosome-linked Mendelian characters (Legro and Strauss, 2002).

The current data strongly support an association between PCOS and the risk of suffering from long-lasting diseases. The pathologies that have been associated wit PCOS include:

- Type 2 diabetes mellitus
- Hypercholesterolemia
- High blood pressure
- Gestational diabetes mellitus
- Hypertension induced during pregnancy (pre-eclampsia, eclampsia)
- Endometrial cancer, and associations between PCOS and breast cancer, ovarian cancer and nuclear cancer have recently been reported.

This emphasizes the need of an early diagnosis of the syndrome and a close follow-up of women with PCOS.

By searching the molecular bases of PCOS, several research groups have started a systematic search of genetic risk factors involved in the predisposition to suffer from PCOS and in its prognosis (Legro and Strauss, 2002). Several genes involved in reproduction, which genes affect the secretion or action of insulin and the genes involved in obesity and energy regulation have been studied as candidate genes. Attention has specifically been focused on genes encoding steroidogenic enzymes of the androgen biosynthesis route (Urbanek *et al.,* 1999; Gharani *et al.,* 1997; Carey *et al.,* 1994) and on the genes involved in the secretion and action of insulin (Urbanek *et al.,* 1999; McKeigue and Wild, 1997; Waterworth *et al.,* 1997; Talbot *et al.,* 1996; Eaves *et al.,* 1999).

PCOS has been associated with a 2-7 times risk of suffering from type 2 diabetes mellitus (T2DM). Previous epidemiological and genetic studies have shown that PCOS and T2DM could share genetic predisposition factors associated with both pathologies. Using this working hypothesis, several studies have suggested that genes related with T2DM could have an important role in the pathogenesis of PCOS (Ehrmann, Tang *et al.,* 2002; Ehrmann, Schwarz *et al.,* 2002; González *et al.,* 2002; Escobar-Morreale *et al.,* 2002). The first gene of predisposition to suffer from type 2 diabetes mellitus disclosed by a wide examination of the genome and positional cloning was a member of the family of cysteine proteases similar to calpain which is expressed ubiquitously as *CALPAIN-10 (CAPN10)* (Horikawa *et al.,* 2000). Association studies using intragenic markers of the *CAPN10* gene has disclosed that different alleles can contribute to the genetic predisposition to suffer from T2DM in several populations (Horikawa *et al.,* 2000; Evans *et al.,* 2001; Garant *et al.,* 2002). Thus, for example, document WO 00/23603 describes the detection of a polymorphism in the *CAPN10* gene associated with the predisposition to suffer from type 2 diabetes mellitus. Although it indicates that the method could be valid to detect polymorphisms in portions encoding other proteases, especially of the calpain family, the search or the detection of another polymorphism that is not the one associated to the *CAPN10* gene is not described and associations with disorders other than type 2 diabetes mellitus are not carried out either. New candidates to risk alleles and genotypes within the *CAPN10* gene, which could be associated with important phenotypic differences observed in patients with PCOS (Ehrmann *et al.,* 2002; González *et al.,* 2003), hypercholesterolemia (Daimon *et al.,* 2002; González *et al.,* 2003), hypertension (Hong *et al.,* 2002), obesity (Shima *et al.,* 2003), and recently with hypertriglyceridemia (Carlsson *et al.,* 2004), have recently been identified. As in the previous case, these works do not show alterations in genes other than *CAPN10* that could also be related to the predisposition to develop PCOS or the mentioned cardiovascular risk factors.

*CAPN5* is a paralogue (a gene homologous to another gene within a same species which has been generated by genetic duplication) of the *CAPN 10* gene which, like the latter, encodes a protease which has been involved in regulating several cell functions, including intracellular signaling, proliferation, differentiation and apoptosis (Ono *et al.,* 1998; Sorimachi *et al.,* 1997; Suzuki *et al.,* 1992). Although the proteases encoded by both *CAPN5* and *CAPN10* tend to be considered as ubiquitous proteins, consulting the NCBI (National Center for Biotechnology Information) website, http://www.ncbi.nim.nih.gov/, shows that there are tissues in which they are not expressed, the expression patterns of the proteins encoded by each gene further being different. The number of different isoforms which they have are also different, 8 isoforms being known for CAPN10 (ranging between 138 and 672 amino acids) and only one for CAPN5 (640 amino acids). Considering the larger CAPN10 isoforms, it is acknowledged that the organization in structural domains of protein sequences encoded by the *CAPN5* and *CAPN10* genes is considerably similar, although the alignment of the protein sequences of *CAPN5* (640 amino acids) and isoform a (672 amino acids) of *CAPN10* shows that only 31% of the amino acids of both proteins are the same and are in the same position. As is logical between paralogues, the gene sequences are considerably homologous, although they differ in length (49,136 bp encoding in *CAPN10,* reaching 59,192 bp in *CAPN5*), in the number of exons and introns (15 exons in the case of *CAPN10,* 13 in the case of *CAPN5*), in the presence of an intragenic gene within intron 3 of the *CAPN5* gene (OMP: olfactory marker protein) and its location in the genome (*CAPN10* is located in chromosome 2, specifically in 2q37.3, whereas *CAPN5* is in chromosome 11, in 11q14). In spite of having similarities with *CAPN10,* the commented state of the art on the latter gene had not suggested nor analyzed a possible association between polymorphisms in the *CAPN5* sequence and the predisposition to suffer from type 2 diabetes mellitus, PCOS or the disorders commonly associated with PCOS. The differences observed between *CAPN10* and *CAPN5* regarding their position in different chromosomes, choir different isoforms, the low percentage of alignment between their amino acid sequences, the different number of exons and introns and, especially a different expression pattern, make it evident to think that *CAPN10* and *CAPN5* have different functions and are involved in different mechanisms or metabolic pathways which in turn can give rise to their involvement in unrelated diseases.

For its part, document WO 02/45491 describes a method for searching for associations between certain proteases (including *CAPN5)* and the predisposition to suffer from certain disorders, but the method is different: generating cell lines and transgenic mice with the modified *CAPN5* gene and observing the possible phenotypic changes produced in them. Although it is suggested that the comparison of the genomic or cDNA sequences of individuals with disorders with the sequences corresponding to healthy individuals could be useful for the detection of possible involved mutations, a specific method or candidate to a mutated gene are not detailed and all the comments on this matter are focused on the case of cancer.

Franz *et al.* (2004) also deals with the study of the consequences that the calpain 5 gene alteration can have in the organism. The method used is again different from that of the invention: the generation of transgenic mice with the modified *CAPN5* gene and the observation of the tissues in which the modified protein is expressed and the possible phenotypic changes generated in the mice. Although a reduction in the viability of transgenic mice is mentioned and no correlations with any specific disorder are established.

By means of genotyping the Finnish population, Silander *et al.* (2004) identified a wide region in chromosome 11 which included genes such as CAPN5 and THRSP, with a high linkage to the predisposition to develop diabetes. Said linkage was not assigned to any specific gene, to any polymorphism within any gene nor to any haplotype related to said polymorphisms.

However there is no disclosure in the state of the art that directly relates the changes in the *CAPN5* gene sequence to the development or the predisposition to develop PCOS in humans, to any of the pathologies with which said syndrome is associated or to pathologies involving cardiovascular risk factors, associated or non-associated to PCOS. The present invention develops a methodology based on the *CAPN5* gene and on the detection of the following polymorphisms in said gene: Nt g.86 A>G, Nt g.344 G>A, Nt c.1320 C>T and Nt c.1469 G>A.


LITERATURE


Balkau B, Charles MA. Comment on the provisional report from the WHO consultation, European Group for the Study of Insulin Resistance (EGIR) Diabet Med. 1999 May; 16(5) : 442-443.

Bonferroni CE (1936). "Teoría statistica delle classi e calcolo delle probabilitá". Pubblicazioni del Istituto Superiore di Scienze Economiche e Commerciali di Firenze, 8:3-62.

Buch B, Galan JJ, Lara M, Ruiz R, Segura C, Real LM, Martinez-Moya M and Ruiz A (2003) Scanning of Y-chromosome azoospermia factors loci using real-time polymerase chain reaction and melting curve analysis. Fertil Steril 80(4),907-913.

Carey AH, Waterworth D, Patel K, White D, Little J, Novelli P, Franks S and Williamson R (1994) Polycystic ovaries and premature male pattern baldness are associated with one allele of the steroid metabolism gene CYP 17. Hum Mol Genet 3,1873-1876.

Carlsson E, Fredriksson J, Groop L, Ridderstrale M. (2004). Variation in the calpain-10 gene is associated with elevated triglyceride levels and reduced adipose tissue messenger ribonucleic acid expression in obese Swedish subjects. J Clin Endocrinol Metab. 89(7):3601-5

Cartegni L, Chew SL, Krainer AR (2002). Listening to silence and understanding nonsense: exonic mutations that affect splicing. Nat Rev Genet 3.285-98.

Cartegni L, Krainer AR (2003). Correction of disease-associated exon skipping by synthetic exon-specific activators. Nat Struct Biol. 10:120-5

Chobanian AV, Bakris GL, Black HR, Cushman WC, Green LA, Izzo JL Jr, Jones DW, Materson BJ, Oparil S, Wright JT Jr, Roccella EJ; Joint National Committee on Prevention, Detection, Evaluation, and Treatment of High Blood Pressure. National Heart, Lung, and Blood Institute; National High Blood Pressure Education Program

Coordinating Committee. Seventh report of the Joint National Cornmittee on Prevention, Detection, Evaluation, and Treatment of High Blood Pressure. Hypertension. Dec 2003;42(6):1206-52. Epub 01 Dec 2003.

Conde L, Vaquerizas JM, Santoyo J, Al.Shahrour F, Ruiz-Llorente S, Robledo M, Dopazo J (2004) Pupa SNP Finder: a web tool for finding SNPs with putative effect at transcriptional level. Nucleic Acids Res. 32:W242-8.

Crosignani PG and Nicolosi AE (2001) Polycystic ovarian disease: heritability and heterogeneity. Hum Reprod Update 7,3-7.

Daimon M, Oizumi T, Saitoh T, Karneda W, Yamaguchi H, Ohnuma H, Igarashi M, Manaba H, Kato T. (2002). Calpain 10 gene polymorphisms are related, not to type 2 diabetes, but to increased serum cholesterol in Japanese. Diabetes Res Clin Pract 15 56:147-52.

Eaves IA, Bennett ST, Forster P, Ferber KM, Ehrmann D, Wilson AJ, Bhattacharyya S, Ziegler AG, Brinkmann B and Todd JA (1999) Transmission ratio distortion at the 1NSIGF2 VNTR. Nat Genet 22,324-325.

Ehrrmann DA, Schwarz PE, Hara M, Tang X, Horikawa Y, Imperial J, Bell GI and Cox NJ (2002) Relationship of calpain-10 genotype co phenotypic features of polycystic ovary syndrome. J Clin Endocrinol Metab 87,1669-1673.

Ehrrmann DA, Tang X, Yoshiuchi I, Cox NJ and Bell GI (2002) Relationship of insulin receptor substrate-1 and -2 genotypes to phenotypic features of polycystic ovary syndrome. J Clin Endocrinol Metab 87,4297-4300.

Escobar-Morreale HF, Peral B, Villuendas G, Calvo RM, Sancho J and San Millán JL (2002) Common single nucleotide polymorphisms in intron 3 of the calpain-10 gene influence hirsutism. Fertil Steril 77,581-587.

Evans JC, Frayling TM, Cassell PG, Saker PJ, Hitman GA, Walker M, Levy JC, O'Rahilly S, Rao PV, Bennett AJ et al. (2001) Studies of association between the gene for calpain-10 and type 2 diabetes mellitus in the United Kingdom. Am J Hum Genet 69,544-552.

Expert Panel on Detection, Evaluation, and Treatment of High Blood Cholesterol in Adults. Executive Summary of The Third Report of The National Cholesterol Education Program (NCEP) Expert Panel on Detection, Evaluation, And Treatment of High Blood Cholesterol 1n Adults (Adult Treatment Panel 111). JAMA. 16 May 2001,285(19):248610 2497.

Fackenthal JD, Cartegni L, Krainer AR, Olopade OI (2002). BRCA2 T2722R is a deleterious allele that causes exon skipping. Am J Hum Genet. 71 :625-31

Garant MJ, Kao WH, Brancati F, Coresh J, Rami TM, Hanis CL, Boerwinkle E and Shuldiner AR (2002) SNP43 of CAPNIO and the risk of type 2 Diabetes in African-Americans: the Atherosclerosis Risk in Communities Study. Diabetes 51,231-237.

Genuth S, Alberti KG, Bennett P, Buse J, Derronzo R, Kahn R, Kitzmiller J, Knowler WC, Lebovitz H, Lernmark A, Nathan D, Palmer J, Rizza R, Saudek C, Shaw J, Steffes M, Stern M, Tuomilehto J, Zimmet P; Expert Committee on the Diagnosis and Classification of Diabetes Mellitus. Follow-up report on the diagnosis of diabetes mellitus. Diabetes Care. Nov 2003Y-26(11):3150-7.

Gharani N, Waterworth DM, Batty S, White D, Gilling-Smith C, Conway GS, McCarthy M, Franks S and Williamson R (1997) Association of the steroid synthesis gene CYPllα with polycystic ovary syndrome and hyperandrogenism. Hum Mol Genet 6,397-402.

González A, Abril E, Roca A, Aragon MJ, Figueroa MJ, Velarde P, Royo JI, Real LM and Ruiz A (2002) CAPN10 alleles are associated with polycystic ovary syndrome. J Clin Endocrinol Metab 87,3971-3976.

González A, Abril E, Roca A, Aragon MJ, Figueroa MJ, Velarde P, Ruiz R, Fayez O, Galan JJ, Herreros JA, Real LM, Ruiz A (2003) Specific CAPN10 gene haplotypes influence the clinical profile of polycystic ovary patients. J Clin Endocrinol Metab 588(11), 5529-5536.

Govind A, Obhrai MS and Clayton RN (1999) polycystic ovaries are inherited as an autosomal dominant trait:

analysis of 29 polycystic ovary syndrome and 10 control families. J Clin Endocrinol Metab 84,38-43.

Haffner SM, Kennedy E, Gonzalez C, Stern MP, Miettinen H. A prospective analysis of the HOMA model. The Mexico City Diabetes Study. Diabetes Care. Oct 1996; 19(10):113 8--41.

Hardy GH (1908). "Mendelian proportions in a mixed population". Science 28;49-50.

Hong J, Li G, Li C, Hui R, Sun S, Wang J, Ye J, Cai H: (2002). Relationship between calpain-10 gene polymorphism, hypertension and plasma glucose]. Zhonghua Nei Ke Za Zhi 41:370-3.

Horikawa Y, Oda N, Cox NJ, Li X, Orho-Melander M, Hara M, Hinokio Y, Lindner TH, Mashima H, Schwarz PE, del Bosque-Plata L et al. (2000) Genetic variation in the gene encoding calpain-10 is associated with type 2 diabetes mellitus. Nat Genet 26,163175.

Kruskal WH and Wallis WA (1952). "Use of ranks in one-criterion variance analysis". J. Amer. Statist. Assoc., 47, 583-621; errata, ibid., 48, 907-911.

Legro RS, Driscoll D, Strauss JF 3rd, Fox J and Dunaif A (1998) Evidence for a genetic basis for hyperandrogenemia in polycystic ovary syndrome. Proc Natl Acad Sci U S A 95,14956-14960.

Legro RS and Strauss JF (2002) Molecular progress in infertility: polycystic ovary syndrome. Fertil Steril 78,569-576.

Lewontin RC and Kojima K (1960) The evolutionary dynamics of complex polymorphisms. Evolution 14,450-472.

McKeigue P and Wild S (1997) Association of insulin gene VNTR polymorphism with polycystic ovary syndrome. Lancet 349,1771-1772.

Ono Y, Sorimachi H and Suzuki K (1998) Structure and physiology of calpain, an enigmatic protease, Biochem Biophys Res Commun 245,289-294.

Real LM, Gayoso AJ, Olivera M, Caruz A, Ruiz A and Gayoso F (2001) Detection of nucleotide c985 A-->G mutation of medium-chain acyl-CoA dehydrogenase gene by real-time PCR. Clin Chem, 47:958-959.

Ronaghi M, Karamohamed S, Pettersson B, Uhlen M, Nyren P. Real-time DNA sequencing using detection of pyropnosphate release. Anal. Biochem. 1996 Nov 1;242(1):84-89.

Sasieni PD (1997) From genotypes to genes: doubling the sample size. Biometrics 53,1253-1261.

Shima Y, Nakanishi K, Odawara M, Kobayashi T, Ohta H (2003). Association of the SNP-19 genotype 22 in the calpain-10 gene with elevated body mass index and hemoglobin Alc levels in Japanese. Clin Chim Acta 336:89-96.

Silander K. et al. (2004). A large set of Finnish affected sibling pair families with type 2 diabetes suggests susceptibility loci on chromosomes 6, 11 and 14. Diabetes 53, 821829.

Sorimachi H, Ishiura S and Suzuki K (1997) Structure and physiological function of calpains. Biochem J 328,721-732.

Suzuki H, Saido TC and Hirai S (1992) Modulation of cellular signals by calpain, Ann N Y Acad Sci 674,218-227.

Talbot JA, Bicknell EJ, Rajkhowa M, Krook A, O'Rahilly S and Clayton RN (1996) Molecular scanning of the insulin receptor gene in women with polycystic ovarian syndrome. J Clin Endocrinol Metab 81,1979-1983.

Tregouet DA, Barbaux S, Escolano S, Tahri N, Golmard JL, Tiret L and Cambien F (2002) Specific haplotypes of the P-selectin gene are associated with myocardial infarction. Hum Mol Genet 11,2015-2023.

Tregouet DA, Barbaux S, Poirier O, Blankenberg S, Bickel C, Escolano S, Ruppc-echt HJ, Meyer J, Cambien F and Tiret L (2003) Gene Polymorphisms in Relation to Plasma SELPLG Levels and Coronary Artery Disease Ann. Hum Genet 67,504-511.

Tregouet DA, Escolano S, Tiret L, Mallet A, Golmard JL. A new algorithm for haplotype-based association analysis: the Stochastic-EM algorithm. Ann Hum Genet. 2004 Mar; 68 (Pt 2):165-77.

Urbanek M, Legro RS, Driscoll DA, Azziz R, Ehrmann DA, Norman RJ, Strauss LII J,F, Spielman RS and Dunaif (1999) Thirty-seven candidate genes for polycystic ovary syndrome: strongest evidence for linkage is with follistatin. Proc Natl Acad Sci U S A 96,8573-8578.

Waterworth DM, Bennett ST, Gharani N, McCarthy MI, Hague S, Batty S, Conway GS, White D, Todd JA, Franks S and Williamson R (1997) Linkage and association of insulin gene VNTR regulatory polymorphism with polycystic ovary syndrome. Lancet 349,986-990.

Weinberg W. (1908). "Über den Nachweis der Vererbung beim Menschen". Jahresh. Verein f. Vaterl. Naturk in Wüttemberg 64:368-82.

Weiss KM and Terwilliger JD (2000) How many diseases does it take to map a gene with SNPs. Nat Genet 26,151-157.

## General Description of the Invention

The object of the present invention is to show the role of *CAPN5*, the gene encoding the CAPN5 protein, in the predisposition to suffer from both PCOS and any of the pathologies considered as cardiovascular risk factors (obesity, diabetes, hypertension, hypercholesterolemia and hypertriglyceridemia), which frequently occur associated to PCOS. To that end, two association studies were carried out by studying the possible relationship between the occurrence of the pathologies and the frequency of four intronic polymorphisms within the *CAPN5* gene (Nt g.86 A>G, Nt g.344 G>A, Nt c.1320 C>T and Nt c.1469 G>A) in the following population groups:

1. In the case of PCOS, a series of patients (148 women affected by PCOS) who showed PCOS, detected by means of ultrasound scans, combined with one of more clinical features of PCOS was used and the frequency of the occurrence of said polymorphisms was compared with normal controls.
2. In the case of cardiovascular risk factors, an intra-cohort study (within the population group itself) was carried out in 606 unrelated individuals (278 men and 328 women), observing in them the presence or absence of fourteen phenotypic characteristics involving cardiovascular risk factors by themselves or as a result of the association thereof (the case of metabolic syndrome) and the possible relationship with the occurrence of the mentioned intronic polymorphisms.
In order to analyze the role of *CAPN5* in the occurrence of these phenotypic features, corresponding haplotype-genotype-phenotype correlation studies of the *CAPN5* gene were carried out in the populations corresponding to each of the two studies (the 148 women affected by PCOS on one hand and the 606 unrelated individuals on the other).
Attempts were also made to locate other *CAPN5* gene variants that might be involved in disorders caused by alterations in the expression of said gene. For this purpose, a new analysis of the available *CAPN5* sequence was carried out with different software, two polymorphisms that could have a deleterious effect on gene expression and contribute to the phenotypic charts of the second study being located. In order to confirm this hypothesis, a subgroup of the population of the study regarding the relationship with cardiovascular risk factors was selected and it was observed if these variants were present in their genome.

**Detailed Description of the Invention**

*Study of the relationship between PCOS and the polymorphisms in the CAPN5 gene*

Patients

The study population consisted of 148 unrelated women with PCOS. All the patients and controls were Caucasian (European white).

According to Homburg, 2002, PCOS was defined by the presence of bilateral polycystic ovaries detected in examinations by means of ultrasound scans: up to a total of fifteen to twenty follicles with a diameter of less than 10 mm between both ovaries, an increase in the stroma volume and an increase in ovary volume (> 9 ml). In addition to said ultrasound scans, the following clinical features were determined: 1) prolonged and idiopathic amenorrhea, defined as the absence of menstruation for at least more than 3 consecutive months (as opposed to normal monthly periodic menstruation or eumenorrhea) and/or oligomenorrhea (menstrual cycles > 35 days, 2) clinical and/or biochemical hyperandrogenism: hirsutism according to Ferriman and Galleway criteria, acne, alopecia, increase in testosterone (T) levels (>3 ng) or 3) anovulatory infertility. In the clinical feature of hirsutism, there are women with PCOS who do not have hirsutism; therefore, both subgroups are distinguished when clinical and biochemical criteria for the assignment of patients with PCOS are used (see Table VII). Patients with the following exclusion criteria were excluded: hyperprolactinemia, thyroid disorders, and nonclassic 21-hydroxylase deficiency (Zawadzky and Dunaif, 1992). To estimate the population frequencies of the SNPs analyzed, 94 non-selected healthy controls of the same race and geographical region were genotyped anonymously.

DNA Extraction

5 ml of peripheral blood were obtained from all patients and controls to isolate germ line DNA from leukocytes. DNA extraction was performed according to standard procedures using NucleoSpin Blood Kit (Macherey-Nagel). DNA aliquots were prepared at a concentration of 5 ng/$\mu$l to perform PCRs. The rest of the stock was cryopreserved at -20 C.

Single Nucleotide Polymorphisms (SNP)

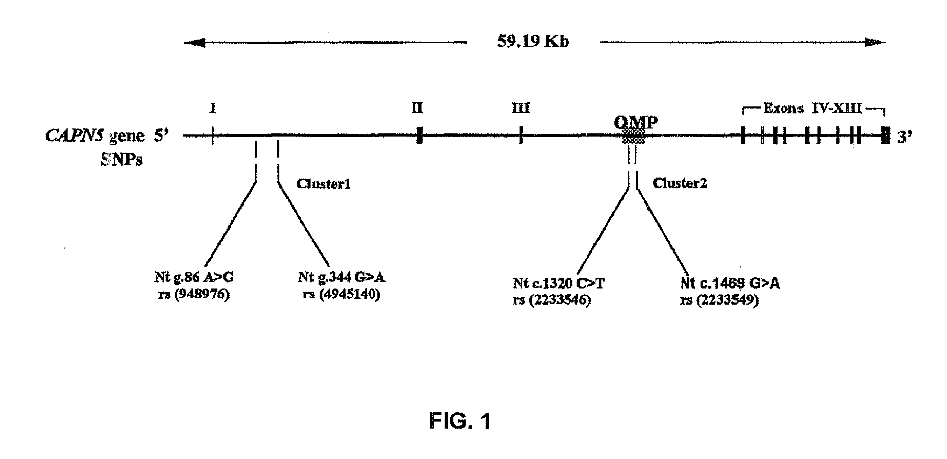

The genomic sequence used to carry out this study corresponds to the genomic sequence of the *CAPN5* gene. The genomic sequence containing the *CAPN5* gene (Contiguous Genomic Segment Group NT 033927, locus ID 726, ENSG00000149260) was identified using the blat tool in the http://www.ucsc.edu webpage, belonging to UCSC (University of California Santa Cruz) with a probe for mRNA of *CAPN5* (GenBank accession number NM004055). The gene includes 59,192 bp comprising 13 exons and 12 introns in 11q.14.
Searching for SNP, germ line DNA from forty unrelated individuals is used, and this gene was traced selecting two candidate areas, if possible defined as clusters (calpain gene regions in which there is a proximity between polymorphisms implying that, in each of these regions, the polymorphisms are not transmitted to offspring independently, but as a single block), located in intron 1 and intron 3 (Figure 1). Four sites of DNA with variations were identified (see Figure 1) using two-way automated DNA sequencing, which sites were organized in two clusters; 1) two polymorphisms located in region 5', intron 1 of the *CAPN5* gene: Nt g.86 A>G and Nt g.344 G>A (according to GenBank accession number AY547311), and 2) two polymorphisms located in the region encoding the OMP (Olfactory Marker Protein) gene, intron 3 of the *CAPN5* gene: Nt c.1320 C>T and Nt c.1469 G>A (according to GenBank accession number U01212). The information regarding the detected markers was compared to the information of the UCSC Genome Bioinformatics and Genome Database (GDB) web pages.

Example 1. Cluster 1 genotyping: Nt g.86 A>G and Nt g.344 G>A

The parallel analysis of these polymorphisms -was genotyped using automated DNA sequencing methods. Amplification primers covering the entire 5' region of the study were designed for the PCR (Table I).

**Table I.** Primers and probes used to genotype the *CAPN5* markers

| **Nt c.1320 C>T and Nt c.1469 G>A** | |
|---|---|
| Primers | OmpF: SEQ ID NO: 1 |
| | OmpR: SEQ ID NO: 2 |
| **Nt c.1320 C>T** | |
| Probes | 46-sensor: SEQ ID NO: 3 |
| | 46-anchor: SEQ ID NO: 4 |
| **Nt c. 1469 G>A** | |
| Probes | 49-sensor: SEQ ID NO: 5 |
| | 49-anchor: SEQ ID NO: 6 |
| **Nt g. 86 A>G and Nt g. 344 G>A** | |
| Primers | capn5F: SEQ ID NO: 7 |
| | capn5R: SEQ ID NO: 8 |

Primers and internal probes used to amplify and detect the genotypes of Nt g.86 A>G, Nt g.344 G>A, Nt c.1320 C>T and Nt c.1469 G>A of the *CAPN5* gene.

PCR was performed with a final volume of 10 $\mu$l using 10 ng of genomic DNA, 1 mM of each amplification primer, 4.4 mM of $MgCl_2$ and 1 $\mu$l of the reaction mixture LC Faststart DNA Master SYBR green I (Roche Applied Science). The amplification conditions during PCR in the Lightcycler thermal cycler were an initial denaturation step at 95°C for 7 minutes, followed by 40 cycles of 95°C for 0 seconds, 67°C for 10 seconds and 72°C for 45 seconds. The PCR products were directly purified and sequenced using the primers Capn5F (SEQ ID NO: 7) and Capn5R (SEQ ID NO: 8). The sequencing reactions were carried out using a CEQ dye terminator cycle sequencing quick start kit (Beckman Coulter, Inc.), according to manufacturer's instructions, and were analyzed with the CEQ™ 8000 genetic analysis system (Beckman Coulter, Inc.).

Example 2: Cluster 2 genotyping: Nt c.1320 C>T and Nt c.1469 G>A

Amplification primers and fluorescent detection probes were designed and synthesized for the PCRs of the *CAPN5* gene using the Web Primer software (genome-www2.stanford/edu.cgi-bin/SGD/web-primer) following the manufacturer's instructions. The selected primer pairs and the detection probes are summarized in Table I. The technique used is called FRET (fluorescence resonance energy transfer).

PCR conditions: Real-time PCR was performed in the LightCycler system (Roche) using previously published reaction conditions (González-Gómez *et al.,* 2003; Real *et al.,* 2001, Buch *et al.,* 2003). PCR was performed to amplify the segments of the *CAPN5* gene flanked by the two polymorphic sites within intron 3. The PCR reactions were carried out in a final volume of 10 $\mu$l using 10 ng of genomic DNA, 0.5 mM of each amplification primer, 4.4 mM $MgCl_2$, 0.5 mM of each detection probe and 1 $\mu$l of LC Faststart DNA Master hybridization probes (Roche). An initial denaturation step of 95°C for 7 minutes, followed by 40 cycles of 95°C for 0 seconds, 68°C for 10 seconds, and 72°C for 40 seconds was used.

Melting curves: the conditions for obtaining optimal melting curves and spectrofluorimetric genotypes were 95°C for 0 seconds, 63°C for 25 seconds, 45°C for 0 seconds and 80°C for 0 seconds (with a temperature transfer speed of 20°C/s in each step, except the last step, in which the temperature transfer speed was 0.1°C/s). In the last step, a continuous fluorometric register was performed (F3/F1 for Nt c.1320 C>T and F2/F1 for Nt c.1469 G>A), fixing the gains of the system at 1, 50, and 50 on channels F1, F2, and F3, respectively. The genotype results using real time-PCR are shown in figure 1. In order to study the specificity of these assays, selected amplicons of different melting patterns were sequenced using an automated DNA sequencer Beckman Coulter CEQ™ 8000 genetic analysis system).

Example 3. Statistical analysis of the genotypes of CAPN5 polymorphisms

In order to compare allele frequencies and genotypes among non-selected patients and controls, $\chi^2$ tests with Yate's correction were carried out using Statcalc software with analysis software (EpiInfo 5.1, Center for Disease Control, Atlanta, Georgia).

Assays adapted from Sasieni (Sasieni, 1997) were used for the statistical analysis of the genotype distribution, Hardy-Weinberg equilibrium deviation assays, or two-point association studies. These calculations were carried out using the on-line resources of the Institute of Human Genetics, Munich, Germany (http://ihg.gsf.de).

The linkage disequilibrium value (D') between the studied genetic markers, the haplotype frequencies and the association analysis based on haplotypes were calculated using the Thesias software available in http://genecanvas.ecgene.net (Tregouet *et al.,* 2002; Tregouet *et al.,* 2003).

The polymorphic allele frequency in four *loci* within the *CAPN5* gene: Nt g.86 A>G, Nt g.344 G>A, Nt c.1320 C>T and Nt c.1469 G>A were analyzed in 148 PCOS cases comparing them with normal controls. The allele frequency in the Spanish population was 0.71 for allele A in Nt g.86 A>G, 0.54 for allele G in Nt g.344 G>A, 0.93 for allele C in Nt c.1320 C>T, 0.81 for allele G in Nt c.1466 G>A. The difference in the frequencies of the alleles in these genetic markers between PCOS and the controls was not statistically significant.

The genotype distribution was also determined for each polymorphism and was compared between women with PCOS and healthy women. The genotype frequencies observed during this study are consistent with the Hardy-Weinberg equilibrium law (p>0.38, Table II). When the genotypes observed in patients with PCOS are compared with those obtained in the controls, the Nt g.86 A>G genotypes do not appear to be differ to a considerable extent in their distribution, although they are significant at the 0.05 level ($\chi^2$= 4.08, p= 0.04, Table II). The analysis of the genotype observed in the patients with PCOS against those obtained in the controls, provides experimental evidence of the involvement of *CAPN5* in the predisposition to suffer from PCOS, suggesting that the Nt g.86 A>G genotype was associated to PCOS in the studied population.

There were no statistically significant differences in the genotype distribution between the patients with PCOS and the controls in the case of the remaining SNPs (Table II).

**Table II.** Analysis of the genotypes of *CAPN5* polymorphisms in patients with PCOS and the Spanish population

| | Genotypes observed in PCOS | | | P Value, $\chi^2$ (HWE)* p Value, $\chi^2$ PCOS against controls** | Genotypes observed in the control population | | | P Value, $\chi^2$ (HWE ) |
|---|---|---|---|---|---|---|---|---|
| Ntg.86 A>G | AA | AG | GG | p=0.38* | AA | AG | GG | p=0.59 |
| | 79 | 51 | 17 | p=0.04**a | 45 | 44 | 5 | |
| Ntg.344 G>A | GG | GA | AA | p=0.89* | GG | GA | AA | p=0.98 |
| | 49 | 68 | 30 | p=0.84** | 28 | 46 | 20 | |
| Ntc.1320 C>T | CC | CT | TT | p=0.85* | CC | CT | TT | p=1 |
| | 129 | 19 | 0 | p=0.97** | 81 | 13 | 0 | |
| Ntc.1469 G>A | GG | GA | AA | P=0.77* | GG | GA | AA | p=0.66 |
| | 91 | 47 | 10 | p=0.57** | 64 | 25 | 5 | |

a, The assay for the association is adapted from Sasieni (1997), assay for heterozygous.

*, $\chi^2$ HWE: The Hardy-Weinberg equilibrium compares the genotypes observed in a population with the ones inferred from the Hardy-Weinberg law algorithm ($p^2+2pq+q^2=1$).

Example 4. Polymorphism linkage assay

Standardized studies of the linkage disequilibrium in pairs (D') were carried out using the Thesias software in order to analyze the degree in which these polymorphisms are in linkage disequilibrium (LD). The "linkage disequilibrium" concept is related to the fact that, when there is proximity between two polymorphisms in a same region, the polymorphisms are not transmitted independently to the offspring, but as a single block. The way to measure the degree in which this joint transmission of two markers occurs is quantified by means of the parameter D', the values of which range between +1 and -1; a value of D' = $\pm 1$ indicates complete linkage disequilibrium, i.e., they are always transmitted together. The sign is positive when the least frequent alleles of each polymorphism are transmitted together and negative when the most frequent allele of one and the least frequent allele of the other are transmitted together (Lewontin and Kojima, 1960).

The results obtained with the study population are shown in Table III.

**Table III.** Standardized LD in pairs (D') for cases with PCOS and controls.

|  | Nt g.344 G>A | Nt c.1320 C>T | Nt c.1469 G>A |
|---|---|---|---|
| Nt g.86 A>G | -1.00(p<0.001) | 0.52(p<0.001) | 0.01 (Ns) |
| Nt g.344 G>A |  | -0.72 (p<0.001) | 0.06 (Ns) |
| Nt c.1320 C>T |  |  | -1.00 (p=0.002) |
| Ns, Non-significant |  |  |  |

The linkage disequilibrium analysis shows that both polymorphisms of cluster 1 (Nt g.86 A>G and Nt g.344 G>A) are in complete LD and on the other hand, both polymorphisms of cluster 2 (Nt c.1320 C>T and Nt c.1469 G>A) are also in complete LD. These two blocks of consistently high LD are separated by an LD interruption interval between physically close polymorphisms (~ 30 kb). The absence of linkage disequilibrium between the two clusters suggests the presence of a recombination *hot spot* between both clusters.

Example 5: Haplotype Association Assay

The construction of haplotypes, formed by SNPs along the four loci within the genomic region of the *CAPN5* gene and the association analysis based on haplotypes were carried out using Thesias software. Only a small proportion of haplotypes really appears when the SNPs are in high LD. In this sense, nine unique haplotypes, marked as A-I between the cases and combined controls, were detected in the analyzed population. Apart from the haplotype having the wild-type allele in each of the polymorphic loci (haplotype D), there were eight haplotypes formed from several combinations and permutations of the variants of the sequence and the wild-type sequence in each *locus* (Table IV). In an overall manner, the allele distribution between patients with PCOS and the controls throughout these none haplotypes was not statistically different ($\chi^2$= 7.77, p=0.45).

**Table IV.** *CAPN5* haplotypes comprising several combinations of variants of a single nucleotide.

| Haplotypes |  | Nt g.86 A>G | Nt g.344 G>A | Nt c.1320 C>T | Nt c.1469 G>A |
|---|---|---|---|---|---|
| A | AACG |  | + |  |  |
| B | AACA |  | + |  | + |
| C | AATG |  | + | + |  |
| D | AGCG |  |  |  |  |
| E | AGCA |  |  |  | + |
| F | AGTG |  |  | + |  |
| G | GGCG | + |  |  |  |
| H | GGCA | + |  |  | + |
| I | GGTG | + |  | + |  |

+, variant present in the *locus*
If blank, wild-type in the *locus*

Using asymptotic studies gave as a result that the haplotype H was over-represented in patients with PCOS when they were compared with healthy women ($\chi^2$ =4.60; OR=2.37, p=0.03, Table V). Haplotype G was infra-represented in the cases when they were compared with the controls, although only a tendency to association was observed ($\chi^2$=3.38; OR=0.59, p=0.06, Table V).

**Table V.** Comparison of the frequencies of *CAPN5* haplotypes, using Thesias software and asymptotic studies, between patients with PCOS and healthy women.

| | Haplotype | Patients with PCOS (n=148) | | Healthy women (n=93) | | Statistical analysis | |
|---|---|---|---|---|---|---|---|
| | | Chromosomes | % | Chromosomes | % | $p^a$ value | $p^b$ value |
| A | AACG | 101 | 34.4 | 67 | 35.6 | Ns 1[c] | |
| B | AACA | 26 | 8.8 | 17 | 9.0 | Ns | Ns |
| C | AATG | 2 | 0.7 | 2 | 1.1 | Ns | Ns |
| D | AGCG | 64 | 21.8 | 35 | 18.6 | Ns | Ns |
| E | AGCA | 13 | 4.4 | 10 | 5.3 | Ns | Ns |
| F | AGTG | 3 | 1 | 3 | 1.6 | Ns | Ns |
| G | GGCG | 43 | 14.6 | 38 | 20.2 | 0.06** | 0.1 |
| H | GGCA | 28 | 9.5 | 8 | 4.3 | 0.03* | 0.08 |
| I | GGTG | 14 | 4.8 | 8 | 4.3 | Ns | Ns |
| | Totals | 294 | 100 | 188 | 100 | | |

Ns, Non-significant, [a], Asymptotic studies; [b], Thesias software; 1[c], Haplotype used as reference, *, $\chi^2$ Pearson standard (degrees of freedom=1); **, $\chi^2$ of the heterozygous assay.

Example 6: Haplotype context assay

A series of haplotype context assays have also been carried out for each of the four polymorphic loci. The effect of the alleles of each polymorphism on the phenotype according to a haplotype context that only differs in the position of the given polymorphism was analyzed. In these comparisons, it was observed that the Nt g.88 A>G polymorphism was only associated with PCOS when it occurred together with the haplotype context (-GCG), haplotypes D-G (OR=0.45, p=0.02, Table VI), and tendency to the association with the haplotype context (-GCA), haplotypes E-H (OR=4.41, p=0.09, Table VI) was observed. The Nt c.1469 G>A polymorphism was only statistically significant with the haplotype context (GGC-), haplotypes G-H (OR=5.47, p=0.04, Table VI). These results showed that there are three groups of markers associated to PCOS and interestingly, it was observed that the four haplotypes included in this group (D, E, G and H) share a common consensus haplotype, -GC-, specific for the *CAPN5* gene, which confers predisposition to suffer from PCOS.

**Table VI.** Possible haplotype contexts in which their allele effect can be investigated for each polymorphism

| Polymorphisms | Haplotype context | Haplotypes | OR | P value |
|---|---|---|---|---|
| Nt g.86 A>G | -GCG | D-G | 0.45 | 0.02 |
| | -GCA | E-H | 4.41 | 0.09 |
| | -GTG | F-I | Ns | Ns |
| Nt g.344 G>A | A - CG | A-D | Ns | Ns |
| | A - CA | B-E | Ns | Ns |
| | A - TG | C-F | Ns | Ns |
| Nt c.1320 C>T | AA - G | A-C | Ns | Ns |
| | AG - G | D-F | Ns | Ns |
| | GC - G | G-I | Ns | Ns |
| Nt c.1469 G>A | AAC- | A-B | Ns | Ns |
| | AGC- | D-E | Ns | Ns |
| | GGC- | G-H | 5.48 | 0.04 |

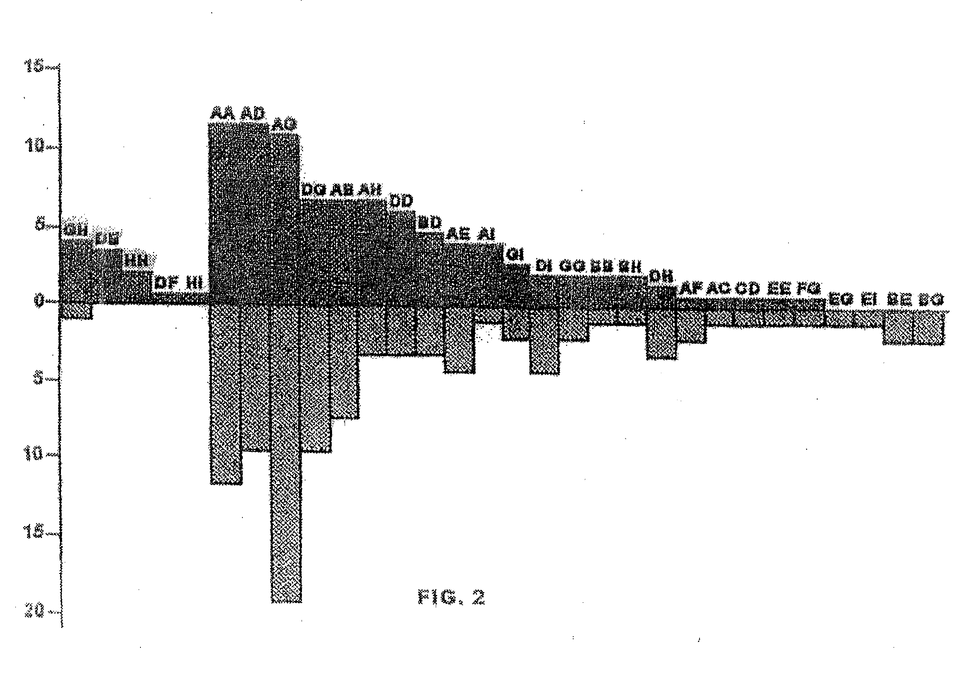

Genotypes comprising pairs of *CAPN5* haplotypes were generated for the cases and the controls and there were 30 different genotypes. The inspection of the genotypes in these two groups showed that the genotypes DE, GH and HH were over-represented among the patients with PCOS, together entailing 10% of the PCOS genotypes, and interestingly, the combinations of haplotypes were only present in a normal control *(x2=7.00,* p=0.008, Figure 2). The four haplotypes involved are again D, E, G and H.

Example 7: Phenotype-genotype correlation assay of clinical symptoms and CAPN5 genotypes

Preliminary-studies were finally carried out to examine the role of the alleles of the *CAPN5* gene in the presence of phenotypic characteristics in patients with PCOS. It was decided to compare the distribution of genotypes and haplotypes of the *CAPN5* gene between groups of patients, divided depending on the presence/absence of specific phenotypic features:

- Menstrual alterations (Amenorrhea/Eumenorrhea -AM/EM-)
- Presence/Absence of Hirsutism (Hirst.*/No Hirst**)
- Severe Complications: Hypercholesterolemia, hypertension, type 2 diabetes mellitus (DM), obesity (the latter measured with a body mass index BMI>25, including pre-obesity conditions) and hypertriglyceridemia (measured as value in blood of triglycerides >150 mg/dl, including cases close to said limit)
- Family history (FH) of cancer (up to second degree of consanguinity = nuclear cancer), essential hypertension, diabetes mellitus (family aggregation), obesity, and other complex diseases related to PCOS as it has been described in the state of the art (Evans *et al.,* 2001).

Table VII shows the results of the phenotype-genotype correlation studies between the SNPs of *CAPN5* and some clinical symptoms related to PCOS. The results indicate that CAPN5 markers have a predictive power to pre-symptomatically infer severe complications in PCOS women with no hirsutism. The presence of FH of obesity, menstrual alterations, the presence of family forms of cancer in PCOS women with hirsutism and the presence of type 2 diabetes mellitus in PCOS women would also have a high correlation with said CAPN5 markers.

**Table VII:** Phenotype-genotype correlation of clinical symptoms and CAPN5 genotypes

| FEATURES | PCOS/Control | Hirst*/No Hirst** | PCOS/Control | Hirst*/No Hirst** | BCOS/Control | Hirs t*/No Hirs t** | PCOS/Control | Hirst*/No Hirst** |
|---|---|---|---|---|---|---|---|---|
| AM/EM | $\chi^2$=4.69 p=0.03 | $\chi^2$=3.79 p=0.05* | - | - | - | - | $\chi^2$=15.31 p=0.0001† | $\chi^2$=15.46 p=0.0001* |
| FH Obesity | $\chi^2$=4.69 p=0.03 | $\chi^2$=7.32 P=0.007* | - | - | - | - | $\chi^2$=6.90 p=0.008 | $\chi^2$=4.38 p=0.03** |
| Cancer | - | - | $\chi^2$=8.61 p=0.003 | $\chi^2$=8.10 p=0.004 | - | - | | |
| FH nuclear cancer | - | - | $\chi^2$=4.97 p=0.03 | $\chi^2$=4.79 p=0.03* | - | - | | |
| Severe complications | _ | - | $\chi^2$=5.26 p=0.02 | $\chi^2$=8.27 p=0.004** | - | - | | |
| DM aggregation | - | - | - | - | $\chi^2$=5.44 p=0.02 | - | | |
| - : non-significant results | | | | | | | | |

***Study of the relationship between pathologies involving cardiovascular risk and polymorphisms in the* CAPN5 *gene***

<u>Study population</u>

The study population consisted of 606 unrelated individuals (278 men, 46%; 328 women, 54%) selected at random. It was determined if each of these individuals showed or did not show one of the following fourteen phenotypic characteristics: 1) obesity, 2) abdominal obesity, 3) systolic hypertension, 4) diastolic hypertension, 5) combined (systolic and diastolic) hypertension, 6) diabetes, 7) glucose intolerance, 8) hyperinsulinemia 9) insulin resistance, 10) hypertriglyceridemia, 11) hypercholesterolemia, 12) low levels of cholesterol associated to high density lipoproteins (hereinafter denominated HDL-cholesterol), 13) high levels of cholesterol associated to low density lipoproteins (hereinafter denominated LDL-cholesterol) and 14) metabolic syndrome, constituting the grouping of some of these factors. The choice of the first three phenotypic characteristics is due to the fact that they are normally considered to be cardiovascular risk factors, which usually occur grouped; thus constituting the so-called metabolic syndrome, phenotypic characteristic number 14 of the study. There are various practical classifications to determine which individuals have the metabolic syndrome, but the most widely used is the Adult Treatment Panel III (ATPIII) of the NCEP (National Cholesterol Education Program, 2001) report, which considers metabolic syndrome to be the presence of at least three of the following cardiovascular risk factors:

a) abdominal obesity
b) hypertriglyceridemia
c) low HDL-cholesterol levels
d) hypertension (systolic and/or diastolic)
e) high fasting glucose

Like the high glucose levels in an oral glucose tolerance test, the latter characteristic is considered to be a glucose intolerance symptom and as such, a step prior to diabetes. Both glucose intolerance and type 2 diabetes mellitus are part of the disorders that can occur as a result of insulin resistance which really is a pathological condition characterized by the absence of the response to insulin by peripheral tissues which means that the tissues cannot recognize it and cannot use it even though there are high blood sugar levels. Therefore, a series of metabolic disorders occur which include dyslipidemia (hypertriglyceridemia, low HDL-cholesterol levels...), abdominal obesity, hyperuricemia, hyperandrogenism etc., in addition to the already mentioned glucose intolerance and type 2 diabetes mellitus. That is why different ways are usually used to assess it. One of them is the homeostatic model (HOMA), used in this study, relating the insulin and glucose blood levels. The specific formula used is:

$$\text{HOMA} = [\text{insulin concentration } (\mu\text{U/ml}) \times \text{glucose concentration } (\text{mmol/l})]/22.5 \text{ the insulin resistance being judged for values equal to greater than that determined by the } 75^{\text{th}} \text{ percentile: HOMA} = 3.67.$$

The reference values used to judge the presence or absence of the other cardiovascular risk factors mentioned also vary between different authors.
In the present study, in an attempt to consider the features associated to diabetes and the steps prior to its development and the summary of alterations constituting the metabolic syndrome, it has been decided to analyze the aforementioned thirteen phenotypic characteristics, as well as the grouping of some of them constituting the metabolic syndrome as such. For each of these characteristics, it was decided whether each particular individual belonged to the case group or the control group in order to carry out the corresponding association study between the calpain-5 gene polymorphisms and this specific phenotypic characteristic. The assignment of each individual to the corresponding case group or the control group was carried out according to the criteria shown in Table VIII, which also shows the distribution of cases and controls obtained for each phenotypic characteristic studied and the references mentioning said criteria are cited.

**Table VIII.** Study population distribution between cases and controls

| PHENOTYPE | NO. OF CONTROLS AND (%) | NO. OF CASES AND (%) | CASE DEFINITION | REFERENCE |
|---|---|---|---|---|
| Obesity | 452 (75.3%) | 148 (24.7%) | BMI[a] $\geq$ 30 | NCEP-ATPIII, 2001 |
| Abdominal obesity | 410 (68.2%) | 191 (31.8%) | Waist circumference : > 102 cm (men) > 88 cm (women) | NCEP-ATPIII, 2001 |
| Systolic hypertension | 409 (68.6%) | 187 (31.4%) | $\geq$ 140 mm Hg or in hypertensive treatment | Chobanian *et al.,* 2003 |
| Diastolic hypertension | 432 (72.5%) | 164 (27.5%) | $\geq$ 90 mm Hg or in hypertensive treatment | Chobanian *et al.,* 2003 |
| Combined (systolic+diastolic) hypertension | 448 (75.2%) | 148 (24.8%) | Systolic $\geq$ 140 mm Hg + Diastolic $\geq$ 90 mm Hg | |
| Diabetes | 519 (91.4%) | 49 (8.6%) | Fasting glucose $\geq$ 126 mg/dl, and/or OGTT[b] $\geq$ 200 mg/dl | Genuth *et al.,* 2003 |
| Glucose intolerance | 433 (76.2%) | 135 (23.8%) | Fasting glucose $\geq$ 100 mg/dl, and/or OGTT[b] $\geq$ 140 mg/dl | Genuth *et al.,* 2003 |
| Hyperinsulinemia | 510 (8.5%) | 90 (15%) | 75th percentile: baseline insulin > 15.77 md/dl | Balkau et *al.,* 2003 |
| Insulin resistance | 287 (51.2%) | 274 (48.8%) | 75th percentile: HOMA $\geq$ 3.67 | Haffner *et al.,* 2003 |
| Hypertriglyceridemia | 489 (86.5%) | 76 (13.5%) | Triglycerides $\geq$ 150 mg/dl | NCEP-ATPIII, 2001 |
| Hypercholesterolemia | 427 (75.6%) | 138 (24.4%) | Total cholesterol $\geq$ 240 mg/dl | NCEP-ATPIII, 2001 |
| Low HDL-cholesterol | 478 (84.6%) | 87 (15.4%) | >40 mg/dl (men) >50 mg/dl (women) | NCEP-ATPIII, 2001 |
| High LDL-cholesterol | 92 (16.3%) | 473 (83.7%) | $\geq$ 100 mg/dl | NCEP-ATPIII, 2001 |
| Metabolic syndrome | 459 (84.2%) | 86 (15.8%) | Three of the following: Abdominal obesity Hypertriglyceridemia Low HDL-cholesterol Hypertension Easting glucose $\geq$ 100 mg/dl | NCEP-ATPIII, 2001 |
| [a]BMI: Body Mass Index, calculated as BMI=weight (kg)/height$^2$ (m$^2$) [b]OGTT: Oral Glucose Tolerance Test. Blood level 120 minutes after an oral overload | | | | |

An association between insulin resistance and the *CAPN5* gene was not detected in the subsequent study of the phenotypic characteristics shown in Table VIII. Therefore, it does not appear in the tables below.

The anthropometric and biochemical data of each individual were collected to carry out the studies. For the qualitative studies, the assignment to the case group or to the control group for each of the phenotypic characteristics studied was carried out according to said collected anthropometric and biochemical parameters, although in said studies, as shown in Table VIII, the individuals who are receiving antihypertensive medication are considered to be hypertensive.

The specific anthropometric and biochemical values corresponding to each individual were also used for the quantitative studies, although in these studies the blood pressure values of the individuals who were receiving antihypertensive medication were eliminated.

An intra-cohort study was carried out in this population to estimate, according to the analyzed phenotypic characteristic, the occurrence frequency in cases and controls of the same SNPs of the *CAPN5* gene studied in the patent specification for which the present specification is an addition.

A new analysis of the available *CAPN5* sequence was also carried out with software different from that used to locate the SNPs of the invention, which allowed locating two additional *CAPN5* variants with the possibility of being deleterious. In order to determine of this was so, a sub-cohort of the study group of 606 individuals which has just been described was selected and it was analyzed if these variables were present in their genomes. The performed studies are described in Example 17 and conclude with negative results. The absence of a relationship between the considered pathologies and the *CAPN5* gene in the latter analyzed population subgroup is a good example of the difficulties involved in determining the gene variations that are really related to diseases, disorders and pathologies, it not being evident that a variant or SNP present in a gene will determine the occurrence of a certain pathology or the predisposition to suffer from it.

DNA Extraction

DNA was obtained by following a procedure similar to the one described in the association study between PCOS and CAPN5: 5 ml of peripheral blood were obtained from all patients and controls to isolate germ line DNA from leukocytes. DNA extraction was performed according to standard procedures using NucleoSpin Blood Kit (Macherey-Nagel). DNA aliquots were prepared at a concentration of 5 ng/$\mu$l to perform PCRs. The rest of the stock was cryopreserved at -20 C.

Single Nucleotide Polymorphisms (SNP). Haplotype-genotype-phenotype correlation

The same four single nucleotide polymorphisms (SNP) of the *CAPM5* gene, the identification of which is described in the section "Single Nucleotide Polymorphisms" in the section of the study of the relationship between PCOS and the *CAPN5* gene polymorphisms, were initially used as polymorphic markers to carry out the study.

The parallel analysis of these polymorphisms was genotyped in the same manner as it was carried out in Example 1 of the section of the study regarding PCOS, as described in Examples 8 and 9 *et seq.,* subsequently carrying out the statistical analysis of the results, as described in Examples 10 to 15.

A haplotype re-analysis of the *CAPN5* gene was subsequently carried out in the study population using a mathematical model different form the one used in Example 12 (Thesias), which reinforced the results obtained with this first mathematical model, even improving the statistical significance of the relationship of some of the haplotypes with some of the phenotypic features analyzed. This re-analysis is described in Example 16.

Example 8: Genotyping of Cluster 1: Nt g.86 A>G and Nt g.344 G>A

As in the case of Example 1, the parallel analysis of these polymorphisms was genotyped using automated DNA sequencing methods. Amplification primers covering the entire 5' region of study were designed for the PCR. Due to the high number of individuals analyzed, new primers were designed (except Capn5F, which was also used in the study described in Example 1) to amplify the polymorphisms of cluster 1 separately, with the aim of obtaining amplicons of approximately 200 bp, the optimal size for the detection in a PSQ™ 96 (Pyrosequencing AB, Uppsala, Sweden). This instrument allows the simultaneous analysis of 96 samples by means of the pyrosequencing technique (Ronaghi M., *et al.,* 1996).

|  | Nt g.86 A>G, |
|---|---|
| Primers | Capn5F: SEQ ID NO: 7 |
|  | CAPN5-76int: SEQ ID NO: 9 |
|  | CAPN5-76R: SEQ ID NO: 10 |

(continued)

Nt g.344 G>A

Primers  CAPN5-140int: SEQ ID NO: 11

CAPN5-R2: SEQ ID NO: 12

CAPN5-Rpi: SEQ ID NO: 13

The PCR reactions were performed with a final volume of 10 μl using 10 ng of genomic DNA.

The Capn5F primer marked with biotin (Capn5F, SEQ ID NO: 7) and an antisense primer (CAPN5-76int, SEQ ID NO: 9) were used for the amplification of the Nt g.86 A>G polymorphism. The PCR conditions were the following: an initial denaturation step of 95°C for 5 minutes, 45 cycles of 95°C - 30 seconds, 68°C - 20 seconds, and 72°C - 30 seconds, and a final extension of 5 min at 72°C. An internal primer for the sequencing reaction was also designed (CAPN5-76R, SEQ ID NO: 10).

The primers for the amplification of the Nt g.344 G>A were CAPN5-140int, SEQ ID NO: 11 and CAPN5-R2, SEQ ID NO: 12, the sense primer (CAPN5-140int) being marked with biotin. The PCR conditions were: 95°C for 5 minutes, 45 cycles of 95°C - 30 seconds, 62°C - 20 seconds, and 72°C - 30 seconds, and a final extension of 5 min at 72°C. A new internal anti-sense primer for the sequencing reaction was also designed (CAPN5-Rpi, SEQ ID NO: 13).

Example 9: Cluster 2 genotyping: Nt c.1320 C>T and Nt c. 1469 G>A

As in Example 2, genotyping of the region of the cluster was carried out by means of PCR amplification and genotyping by means of the so-called FRET technique, using the amplification primers and fluorescent detection probes indicated in said Example 2 (see Table 1).

The conditions for carrying out PCR and for obtaining the melting curves were identical to those described in Example 2.

Example 10: Statistical analysis of the genotypes of the *CAPN5* polymorphisms

Similar to the description in Example 3, the comparisons between allele frequencies and genotypes among non-selected patients and controls was carried out by means of $\chi^2$ tests with Yate's correction using Statcal software with analysis software (EpiInfo 5.1, Center for Disease Control, Atlanta Georgia).

Tests adapted from Sasieni (Sasieni, 1997) were used again for the statistical analysis of the genotype distribution, Hardy-Weinberg equilibrium deviation tests or two-point association studies were used, the on-line resources of the Human Genetic Institute, Munich, Germany (http://ihg.gsf.de) being used to carry out the calculations.

Polymorphic allele frequency was analyzed in the 606 individuals of the study at the four loci in the CPN5 gene: Nucleotide g.86 A>G, Nt g.86 A>G, Nt g.344 G>A, Nt c.1320 C>T and Nt c. 1496 G>A. The genotypes and allele frequencies obtained are summarized in Table IX.

Table IX - Analysis of the genotypes of the CAPN5 polymorphisms in the study population

| POLYMORPHISM | GENOTYPES | | | ALLELE f 1* | p H-W** |
|---|---|---|---|---|---|
| Nt g.86 A>G | AA | AG | GG | 0.74 | 0.397 |
| | 338 | 224 | 77 | | |
| Nt g.344 G>A | GG | GA | AA | 0.57 | 0.456 |
| | 200 | 288 | 118 | | |
| Nt c.1320 C>T | CC | CT | TT | 0.94 | 1.0 |
| | 536 | 68 | 2 | | |

(continued)

| POLYMORPHISM | GENOTYPES | | | ALLELE f 1* | p H-W** |
|---|---|---|---|---|---|
| Nt c.1469 G>A | GG | GA | AA | 0.80 | 0.132 |
| | 391 | 184 | 31 | | |

*f: allele frequency
**p H-W: statistical significance obtained in the $\chi^2$ analysis of the Hardy-Weinberg equilibrium (comparison between the obtained allele frequencies and the expected allele frequencies according to algorithm $p^2+2pq+q^2=1$ which defines said equilibrium).

The existence of association between thirteen of the phenotypic characteristics to be analyzed: 1) obesity, 2) abdominal obesity, 3) systolic hypertension, 4) diastolic hypertension, 5) combined (systolic and diastolic) hypertension, 6) diabetes, 7) glucose intolerance, 8) hyperinsulinemia, 9) insulin resistance, 10) hypertriglyceridemia, 11) hypercholesterolemia, 12) low HDL cholesterol levels, 13) high LDL cholesterol levels, was qualitatively analyzed, and the four polymorphisms were individually analyzed qualitatively using the $\chi^2$ analysis adapted from Sasieni (1997). The results are shown in Table X, which indicates only the most significant value of each analyzed phenotype in which an association was found, indicating the corresponding values of p, the odds ratio and the confidence interval (CI).

**Table X**. Association between phenotypes and individual polymorphisms.

| PHENOTYPE | MARKER | P | ODDS RATIO (CI) |
|---|---|---|---|
| Hypercholesterolemia (Total cholesterol > 240) | Nt c.1469 G>A | 0.00512* | 0.331 (0.417-0.743) |
| High LDL cholesterol | Nt g.86 A>G | 0.01469* | 0.297 (0.106-0.832) |
| High LDL cholesterol | Nt c.1320 C>T | 0.00147** | 3.802 (1.581-9143) |
| Combined hypertension | Nt c.1320 C>T | 0.01317*** | 15.506 (0.740-325.08) |
| Combined hypertension | Nt c.1469 G>A | 0.01110** | 0.365 (0.165-0.811) |
| Diastolic hypertension | Nt c.1320 C>T | 0.02150* | 0.075 (0.004-1.574) |

*Positive allele assay for allele 1 (individuals homozygous for allele 2 compared to the group of individuals carrying allele 1)
**Allele 2 heterozygote assay (individuals homozygous for allele 1 compared to homozygous)
***Allele 2 monozygote assay (individuals homozygous for allele 1 compared to homozygous for allele 2)

There are only two possible alleles in the analyzed polymorphisms: 1, usually corresponding to the most frequent variant, and 2, the less frequent one. Given that there are two copies of each of the genes, if the genotype of each individual is considered, it the individual can be homozygous for this polymorphism and have two equal alleles 1 (individual 11) or two alleles 2 (individual 22) or be heterozygous and have one allele of each type (genotype 12). When expressing 11 + 12 vs. 22, the intent is that the analysis has compared the individuals 22 (allele 2 homozygotes) to the group of individuals carrying allele 1 (individuals 11 and 12).

Example 11: Polymorphism linkage assay

To analyze the degree to which these polymorphisms are in linkage disequilibrium (LD), standardized experiments concerning the linkage disequilibrium in pairs (D') were carried out using Thesias software. The results are shown in Table XI and coincide with those obtained in Example 4 when a similar analysis was carried out with the population used for the study referring to PCOS. It was observed that the polymorphisms of Nt g.86 A>G and Nt c.1320 C>T are in complete linkage disequilibrium with the polymorphisms of Nt g.344 G>A and Nt c.1469 G>A, respectively (D'=-1, p<0.0001; D'=-1, p<0.0001, respectively), and between the polymorphisms of Nt g.344 G>A and Nt c.1320 C>T there is an absence of linkage disequilibrium (D'=0.00, p<0.001).

**Table XI. Standardized** LD in pairs (D')

| | Nt g.344 G>A | Nt c.1320 C>T | Nt c.1469 G>A |
|---|---|---|---|
| Nt g.86 A>G | -1.00 (p<0.0001) | -0.03 (p=0.898) | -0.29 (p=0.006) |
| Nt g.344 G>A | | 0.00 (p=0.970) | 0.32 (p<0.0001) |

(continued)

| | Nt g.344 G>A | Nt c.1320 C>T | Nt c.1469 G>A |
|---|---|---|---|
| Nt c.1320 C>T | | | -1.00 (p<0.0001) |

As in the study referring to PCOS, the analysis of the linkage disequilibrium again confirmed the existence of complete linkage disequilibrium in each cluster 1 (Nt g.86 A>G and Nt g.344 G>A) are in complete LD, and on the other hand both polymorphisms of cluster 2 (Nt c.1320 t and Nt c.1469 G>A) are also in complete LD, whereas there is an absence of linkage disequilibrium between the two clusters, again suggesting the presence of a recombination "hot spot" between both clusters.

Example 12; Haplotype association assay

The construction of haplotypes, which are made up of SNPs along the four loci inside the genome region of the *CAPN5* gene and the association analysis based on the haplotypes was again carried out using Thesias software.
As occurred in Example 5 regarding the relationship between PCOS and the *CAPN5* gene, nine unique haplotypes were detected again in the analyzed population, marked as A-I between the cases and combined controls. In addition to the haplotype of the wild-type allele in each of the polymorphic loci (haplotype D), there were eight haplotypes consisting of several combinations and permutations of variants of the sequence and the wild-type sequence in each locus. Table XII shows the identified haplotypes and their frequencies.

**Table XII.-** Identified *CAPN5* haplotypes comprising several combinations of variants of a single nucleotide

| | | Nt g.86 A>G | Nt g.344 G>A | Nt c.1320 C>T | Nt c.1469 G>A | Frequency |
|---|---|---|---|---|---|---|
| A | AACG | | + | | | 0.285352 |
| B | AACA | | + | | + | 0.120482 |
| C | AATG | | + | + | | 0.025833 |
| D | AGCG | | | | | 0.252580 |
| E | AGCA | | | | + | 0.039822 |
| F | AGTG | | | + | | 0.018431 |
| G | GGCG | + | | | | 0.198734 |
| H | GGCA | + | | | + | 0.043029 |
| I | GGTG | + | | + | | 0.015737 |
| + variant present in the *locus* If blank, wild-type in the *locus.* | | | | | | |

Example 13: Haplotype context assay: qualitative analysis

For the purpose of discovering not only which polymorphisms are associated to a certain phenotype but also which is the specific genetic context in which this association occurs, a series of haplotype context assays were carried out for each of the four polymorphic *loci*. The variables were analyzed qualitatively (according to the criteria mentioned in the section in which the "Study population" was described) and quantitatively, this latter case being subsequently shown in Example 14. The level of statistical significance is set at $p=0.05$ (Bonferroni correction is not applicable). The result in the statistical analysis is usually expressed with the value of p, which is simply a way of quantifying the probability with which the observation that is made is true and is not random. Statistical significance is usually established when the value of p obtained is less than 0.05, i.e. the association between a certain genotype and a disease is true with 95% probability. In studies in which several polymorphisms are examined at the same time, it is necessary to make a correction (Bonferroni correction) because each individual analysis presents a risk of false positives. The Bonferroni correction consists of dividing the vale of 0.05 by the number of analyzed polymorphisms. In this case, there are four analyzed polymorphisms, but they are divided by two because they are not completely independent, but rather are linkage disequilibrium in twos. This correction is made only in genotype studies; this correction is not established in haplotype studies because the Thesias program that has been used already establishes its own system of correcting possible errors or false positives, therefore the level of significance is set at 0.05. The lower the value of p the stronger is the association being studies, i.e. the fewer probabilities there are that what is observed is random. The values approaching the statistical significance limit without reaching it are said to show a trend, even more so the closer they are to said values.

Continuous variables are analyzed during quantitative analysis, i.e. variables which can have any value, such as age or the blood sugar level. For this type of variables, the means (the mean value of all the values analyzed so it is necessary that the samples be distributed normally) or medians (the central value of a series when the values are not distributed normally, as in this case) are compared in each genotype and they are analyzed to find if there are significant differences between them.

In qualitative analysis, however, variables which are distributed in categories, for example diabetic and non-diabetic, are compared. When the condition of healthy or afflicted depends on levels of continuous biochemical parameters such as diabetes or hypercholesterolemia, a reference value marking the limit between them is set. The same occurs with almost all the variables that have been analyzed. The reference values used in this study are hereinbefore mentioned at the beginning of the section of the Detailed Description of the Improvements of the Invention and are summarized in Table 1.

By assigning each investigational group with the condition of healthy (control) or afflicted (case) in qualitative analysis, statistical significance can be lost and the results may vary according to the criterion that is used, therefore quantitative studies are preferred. However, as will be discussed below, no discrepancies between both study groups arose, there is just greater sensitivity of the quantitative method.

The association between calpain 5 and obesity, diabetes, glucose intolerance, high LDL cholesterol and metabolic syndrome has been established by means of qualitative analysis. Table XIII includes the statistically significant results. The values of p in each haplotype or genetic context and between parentheses, the OR (odds ratio). The odds ratio values reflect the probability of an individual carrier of a certain allele of suffering the disease and an OR value <1 indicates protection against the disease (less probability of suffering it).

**Table XIII.-** Quantitative analysis of haplotypes

| | | OBESITY (BMI>30) | DIABETES | GLUCOSE INTOLERANCE | LDL CHOLESTEROL | METABOLIC SYNDROME |
|---|---|---|---|---|---|---|
| STATISTICALLY SIGNIFICANT HAPLOTYPES | | **AACA** 0.035596 (OR=1.64175) **AGCA** 0.049591 (OR=0.23349) | **AGCA** 0.048263 (OR=3.11211) | **AGCA** 0.036786 (OR=2.33392) | **GGCG** 0.040231 (OR=0.57398) | **AACA** 0.024481 (OR=1.92605) **AGCG** 0.040914 (OR=0.167511) |
| GENETIC CONTEXT | **Nt g.86 A>G** | | | | | |
| | -GCG | Ns | Ns | Ns | Ns | Ns |
| | -GCA | 0.045292 (OR=5.58645) | Ns | Ns | Ns | Ns |
| | -GTG | Ns | Ns | Ns | Ns | Ns |
| | **Ntg.344 G>A** | | | | | |
| | A-CG | Ns | Ns | Ns | Ns | 0.040914 (OR=0.59698) |
| | A-CA | 0.010567 (OR=7.03145) | Ns | Ns | Ns | Ns |
| | A-TG | Ns | Ns | Ns | Ns | Ns |
| | **Nt c. 1320 C>T** | | | | | |
| | AG-G | Ns | Ns | Ns | Ns | Ns |

(continued)

| | | OBESITY (BMI>30) | DIABETES | GLUCOSE INTOLERANCE | LDL CHOLESTEROL | METABOLIC SYNDROME |
|---|---|---|---|---|---|---|
| | AA-G | Ns | Ns | Ns | Ns | Ns |
| | GG-G | Ns | Ns | Ns | Ns | Ns |
| **Nt c. 1469 G>A** | | | | | | |
| | AGC- | 0.032225 (OR=0.19006) | Ns | Ns | Ns | Ns |
| | AAC- | 0.035596 (OR=1.64175) | Ns | Ns | Ns | 0.024481 (OR=1.92605) |
| | GGC- | Ns | Ns | Ns | Ns | Ns |
| Ns: not significant | | | | | | |

Example 14: Haplotype context assay: quantitative analysis

The quantitative studies have confirmed the association of CAPN5 with the increase of glucose levels in general, LDL cholesterol, to a certain extent, with obesity, but in this case not through the BMI but through the waist circumference measurement, a parameter associated to cardiovascular risk. Statistically significant values were also obtained for diastolic pressure, total cholesterol, low HDL cholesterol levels and high baseline insulin levels.

Table XIV shows the results with statistical significance obtained in the quantitative analysis. The arithmetic mean obtained for said parameter in the study population from the values corresponding to each individual is indicated immediately under the heading corresponding to each phenotype characteristic studied. The characteristics shown are: waist circumference (WC), diastolic pressure (DP), fasting glucose (FG), glucose levels detected in an oral glucose tolerance test (OGTT), baseline insulin (BI), total cholesterol (CHOL), HDL cholesterol (HDL) and LDL cholesterol (LDL). The value of p corresponding to a certain genetic context, e, immediately under it, the effect of the haplotype in question on levels of the phenotype variant (the increase or decrease of the value of the parameter with respect to the mean value of said parameter previously indicated) is indicated for each of the parameters. The values in italics are those values which do not reach statistical significance ($p < 0.05$) but which show a strong trend, therefore they have been included in said Table XIV.

**Table XIV.-** Quantitative Analysis of Haplotypes

| | | WC ½=90.15 | DP ½=76.36 | FG ½=90.03 | OGTT ½=112.6 | BI ½=12.79 | CHOL ½=212.5 | HDL ½=60.67 | LDL ½=131.8 |
|---|---|---|---|---|---|---|---|---|---|
| STATITICALLY SIGNIFICANT HAPLOTYPES | | AGCA 0.0088053 (-5.956) | **AGCA** 0.000971 (-4.505) **GGCG** 0.016729 (-2.802) | **AGCA** 0.034756 (+8.007) | **AGCA** 0.0035222 (+14.381) | **GGTG** 0.0083 (+4.851) | **GGCA** 0.022775 (+18.783) GGTG 0.017729 (+26.358) | **AGCA** 0.006022 (+6.832) | **GGTG** 0.0014348 (+20.847) |
| GENETIC CONTEXT | **Nt g.86 A>G** | | | | | | | | |
| | -GCG | 0.035799 (-2.206) | 0.026427 (-1.900) | Ns | Ns | Ns | Ns | Ns | Ns |
| | -GCA | Ns | 0.010859 (+6.548) | Ns | 0.043761 (-30.53) | Ns | Ns | Ns | Ns |
| | -GTG | Ns | Ns | Ns | Ns | Ns | Ns | Ns | Ns |
| | **Nt g.344 G>A** | | | | | | | | |
| | A-CG | Ns | Ns | Ns | Ns | Ns | Ns | Ns | Ns |
| | A-CA | 0.013785 (+6.139) | 0.008425 (+4.319) | Ns | 0.005620 (+22.847) | Ns | Ns | *0.066531 (-5.774)* | Ns |
| | A-TG | Ns | Ns | Ns | Ns | Ns | Ns | Ns | Ns |
| | **Nt c.1320 C>T** | | | | | | | | |
| | AG-G | Ns | Ns | Ns | Ns | Ns | Ns | Ns | Ns |
| | AA-G | Ns | Ns | Ns | Ns | Ns | Ns | Ns | Ns |
| | GG-G | Ns | Ns | Ns | Ns | 0.0059 (+5.340) | 0.003735 (+31.730) | Ns | 0.002982 (+25.488) |
| | **Nt c.1469 G>A** | | | | | | | | |
| | AGC- | 0.007854 (-6.380) | 0.003205 (-4.323) | *0.062322 (+9.614)* | 0.007204 (+20.043) | Ns | Ns | 0.005475 (+7.775) | Ns |
| | AAC- | Ns | Ns | Ns | Ns | Ns | Ns | Ns | Ns |

| | | WC ½=90.15 | DP ½=76.36 | FG ½=90.03 | OGTT ½=112.6 | BI ½=12.79 | CHOL ½=212.5 | HDL ½=60.67 | LDL ½=131.8 |
|---|---|---|---|---|---|---|---|---|---|
| | GGC- | Ns | 0.39809 (+4.425) | 0.081452 (+7.469) | Ns | Ns | 0.006106 (+24.154) | Ns | 0.069637 (+13.738) |

As previously discussed, the analysis of the results shown in Table XIV and their comparison with the results obtained in the qualitative analysis shows that there is no difference between the quantitative and quantitative analyses, simply greater sensitivity of the quantitative method. Therefore, if an association between CAPN5 and obesity is obtained in the qualitative analysis, an association between CAPN5 and a larger waist circumference (which is another way to determine obesity, abdominal obesity in this case) is obtained in the quantitative analysis. In relation to glucose intolerance, a characteristic associated with CAPN5 according to the qualitative study, the condition of afflicted (the assignment to the case group) is established in those individuals with baseline blood sugar levels exceeding 100 mg/dl and/or an oral glucose tolerance test (OGTT) exceeding 140 mg/dl; the latter parameter has obtained statistical significance in the quantitative analysis. Finally, statistic significance was obtained in both studies in the case of LDL cholesterol.

Example 15: Overall analysis of the study of association between cardiovascular risk pathologies and polymorphisms in the *CAPN5* gene

As an overall result of the haplotype study, it can be seen that most haplotypes associated to the various phenotypes analyzed share the haplotype contexts AGC_ and GGC_. The presence of the latter polymorphism (Nt c. 1469 G>A) has important effects on the levels of the studied variable according to the haplotype context in which it is located. Table XV allows observing this fact more clearly.

**Table XV.**- Effect of polymorphism Nt c.1469 G>A on the phenotype according to the genetic context.

| CONTEXT | P | FEATURE | MEAN VALUE | EFFECT ON VALUE OF THE VARIABLE |
|---|---|---|---|---|
| GGC- | 0.039809 | Diastolic pressure | 76.35 | +4.12538 |
| GGC- | 0.069637 | LDL cholesterol | 131.83 | +13.73889 |
| GGC- | 0.006106 | Total cholesterol | 212.55 | +24.15431 |
| AGC- | 0.007854 | Waist obesity | 90.15 | -6.38013 |
| AGC- | 0.003205 | Diastolic pressure | 76.35 | -4.32349 |
| AGC- | 0.005475 | HDL cholesterol | 60.67 | +7.77562 |
| AGC- | 0.007204 | Glucose tolerance | 112.57 | +20.04337 |

The presence of polymorphism Nt c.1469 G>A in the GGC-context is related to values exceeding the mean in terms of diastolic pressure, total cholesterol and LDL cholesterol, although the latter does not reach the level of statistical significance. It is located in the AGC- context, where a decrease in abdominal obesity (related to cardiovascular risk) and of diastolic pressure occur, further causing an increase in HDL cholesterol levels. It can also be seen that its presence in AGC- (haplotype AGCA) is related to higher glucose levels in the Oral Glucose Tolerance Test (OGTT or glucose after 120 minutes).

Other haplotypes have also shown an association with some alterations. In that sense, haplotype AACA has been associated to obesity and metabolic syndrome, whereas haplotype GGTG is associated to hyperinsulinemia and high total and LDL cholesterol levels.

Example 16: Re-analysis of the *CAPN5* gene using association tests based on logistic regressions

To confirm the previous data, a re-analysis of the haplotype of the CAPN5 gene in the same study population was carried out using a mathematical model that was different from the one used by Thesias, implemented in Whap software (http://www.genome.wi.mit.edu/~shaun/whap/). Thesias uses the SEM algorithm for the interference of the haplotypes and bases the association analysis on differences between the phenotype means associated to each haplotype. Whap uses the EM algorithm (the SEM algorithm being a variant of the EM algorithm) for the interference of haplotypes, but it bases the association analysis on a logistic regression.

According to the software instructions, for greater method precision the values of each series must be standardized such that the mean is equal to 0 and variance equal to 1. These standardized values are obtained subtracting from each value the mean of the series and dividing by the standard deviation thereof $(X-X_{1/2})$ /SD).

A test has been included in the association studies with 5000 permutations to obtain the empirical values of p. The empirical values of p are obtained after analyzing a permutation test. Permutations are the different possible ways of arranging the variables under study. The permutations test randomly distributes the values of the quantitative variable under study and the genotypes the specified number of times (5,000 in this case). The situation in which there are no differences is thus simulated given that the assignment of each value to each genotype is random in each permutation, a reduction of the rate of false positives being obtained. The significant results thus obtained are summarized in Table XVI. The column entitled "Haplotype (1 d.f.)" refers to the value of p or statistical significance obtained by the specific haplotype specified in each case in a $\chi^2$ analysis with one degree of freedom (1 d.f.). The values specified in the column "Overall (5.d.f.)" are general estimates of the extent to which the *CAPN5* haplotypes affect the phenotype under study, without specifying which specific haplotype or haplotypes exert this action. Given that only the six *CAPN5* haplotypes with a frequency exceeding 3% have been used in this analysis, a $\chi^2$ test with five degrees of freedom has been used. Finally, the presence of the asterisk indicates the associations that were significant according to Thesias software.

**Table XVI.-** Study of association between CAPN5 haplotypes and cardiovascular risk pathologies based on logistic regressions (Whap software).

| Phenotype | Haplotype | Haplotype (1 d.f.) | | | Overall (5 d.f.) | | |
|---|---|---|---|---|---|---|---|
| | | $\chi^2$ | p | empirical p | $\chi^2$ | p | empirical p |
| BMI | AACA | 5.32 | 0.021 | 0.016 | 10.78 | 0.056 | 0.037 |
| Waist circumference | AGCA* | 4.13 | 0.042 | 0.034 | 9.06 | 0.107 | 0.122 |
| Diastolic blood pressure | GGCG* | 6.89 | 0.009 | 0.006 | 19.02 | 0.002 | 0.008 |
| | AGAGCA* | 5.23 | 0.022 | 0.034 | | | |
| | AACG | 6.32 | 0.012 | 0.014 | | | |
| Baseline glucose | AGCA* | 5.69 | 0.017 | 0.064 | 8.37 | 0.137 | 0.159 |
| Baseline cholesterol | AGCA* | 5.69 | 0.017 | 0.064 | 8.37 | 0.137 | 0.159 |
| HDL cholesterol | AGCA* | 7.07 | 0.008 | 0.008 | 9.63 | 0.086 | 0.108 |
| | AACA | 4.04 | 0.044 | 0.032 | | | |
| LDL cholesterol | GGCA* | 4.86 | 0.027 | 0.036 | 7.41 | 0.192 | 0.217 |

The analysis of haplotypes has also been carried out taking into consideration the effect of age and sex as covariables. Given that age and sex affect a number of biochemical and anthropometric values, it is common practice in statistics to correct studies of association due to the effect of these variables, because they may be factors which cause confusion altering the genotype-phenotype relationship. The software used for this new study, Whap, allows including these or other factors in the mathematical model which may affect the analysis of association between CAPN5 and the variable under study; these factors are referred to as covariables. Corrected values of association shown in Table XVII have been obtained in this manner. The table shows that the values of p are not substantially modified for any of the phenotypes analyzed, the associations with baseline glucose and total cholesterol being those which take on greater statistical significance with respect to the non-covariate analysis.

**Table XVII.-** Analysis of CAPN5 haplotypes based on logistic regressions taking age and sex into consideration as covariables

| Phenotype | Haplotype | Haplotype (1 d.f.) | | | Overall (5 d.f.) | | |
|---|---|---|---|---|---|---|---|
| | | $\chi^2$ | P | empirical P | $\chi^2$ | P | empirical P |
| BMI | AACA | 4.35 | 0.037 | 0.044 | 10.99 | 0.051 | 0.055 |
| Waist circumference | AGCA* | 1.29 | 0.256 | 0.273 | 7.56 | 0.182 | 0.203 |
| Diastolic blood pressure | GGCG | 3.90 | 0.048 | 0.058 | 11.62 | 0.04 | 0.056 |
| | AGCA* | 3.9 | 0.048 | 0.049 | | | |
| | AACG | 3.62 | 0.057 | 0.069 | | | |

(continued)

| Phenotype | Haplotype | Haplotype (1 d.f.) | | | Overall (5 d.f.) | | |
|---|---|---|---|---|---|---|---|
| | | $\chi^2$ | P | empirical P | $\chi^2$ | P | empirical P |
| Baseline glucose | AGCA* | 7.96 | 0.005 | 0.046 | 9.08 | 0.106 | 0.117 |
| Baseline cholesterol | AGCA | 7.67 | 0.005 | 0.006 | 7.1 | 0.213 | 0.246 |
| HDL cholesterol | AGCA | 3.45 | 0.063 | 0.054 | 8.6 | 0.126 | 0.111 |
| | AACA | 4.04 | 4.917 | 0.026 | | | |
| LDL cholesterol | GGCA | 6.33 | 0.012 | 0.014 | 6.14 | 0.293 | 0.321 |

The results are generally consistent with those obtained by using the SEM algorithm. Furthermore, in the case of obesity in which a p with a marginal significance level was obtained in the quantitative analysis using the Thesias method (0.056), the statistical significance is improved with this other analysis (p=0.037, Table XVII), clarifying the relationship of the gene to obesity.

It can therefore be concluded that the group of symptoms with which the variants of calpain 5 are related are similar to those of the metabolic syndrome, characterized by the presence of three or more of the following factors: obesity, hypertension, glucose intolerance/insulin resistance, hypercholesterolemia and hypertriglyceridemia. Some of these haplotype variants specifically seem to be associated with the predisposition to manifesting metabolic syndrome in the analyzed population. The relationship of *CAPN5* to phenotypes of the metabolic syndrome is resistant to different statistical models, analysis of covariables and use of medication (antihypertensive agents, oral antidiabetics and hypocholesterolemic agents), showing the value of *CAPN5* in the prediction, prognosis and assistance in the diagnosis of diseases that today are as common as hypertension, hypercholesterolemia or obesity. Each risk group may be multifactorial, such that it may be the combination of several of the haplotype variants of the *CAPN5* gene for which the association with cardiovascular risk factors has been detected in other sub-groups, which may be useful in detecting the existence or predisposition of suffering metabolic syndrome.

### *Study of the relationship between pathologies involving cardiovascular risk and polymorphism in the* CAPN5 *gene*

Attempts were made to locate other variants of the CAPN5 gene that may be involved in disorders caused by the altered expression of said gene. For this purpose a new analysis of the available *CAPN5* sequence (ENSG00000149260) was carried out, this time using pupaSNP Finder software (Conde L. *et al.* 2004; http://pupasnp.bioinfo.cnio.es/), different from the one that previously allowed detecting the four previously described SNPs. This software is especially indicated for detecting the variations that may affect messenger RNA splicing. This process consists of a digestion and ligation reaction of the RNA molecules, which must occur before passing to the cytoplasm to be translated to a protein. For this reason, any alteration in the signals controlling this process entails serious consequences in the gene expression.

This new analysis clearly showed two candidate polymorphisms which could have an effect on the expression of CAPN5 due to a possible alteration in the messenger RNA splicing process.

Once exon 2 was located, rs11825526 was found, which is an A>G substitute which broke the structure of an exonic splicing enhancer (ESE), which could entail elimination of the exon. Table XVIII shows how the T>C change alters the action targets (ESEs) of splicing factors srp40 and sf2. ESEs are regulating regions which are found frequently, if not in all, exons, also including those which are constitutional (Cartegni L *et al.,* 2002). To date, many polymorphisms located in encoding regions have been related with altered gene expression patterns. In fact, 50% of the mutations involving an amino acid change and which have been associated to said pathologies cause aberrant splicing (Cartegni L *et al.,* 2003). The literature mentions some interesting examples of this type of mutations (Fackenthal JD *et al.,* 2002). For this reasons it was considered that rs11825526 could be associated to the H and/or W haplotypes and thus be generating an aberrant gene expression pattern and finally contribute to the development of the phenotypic charts of the study.

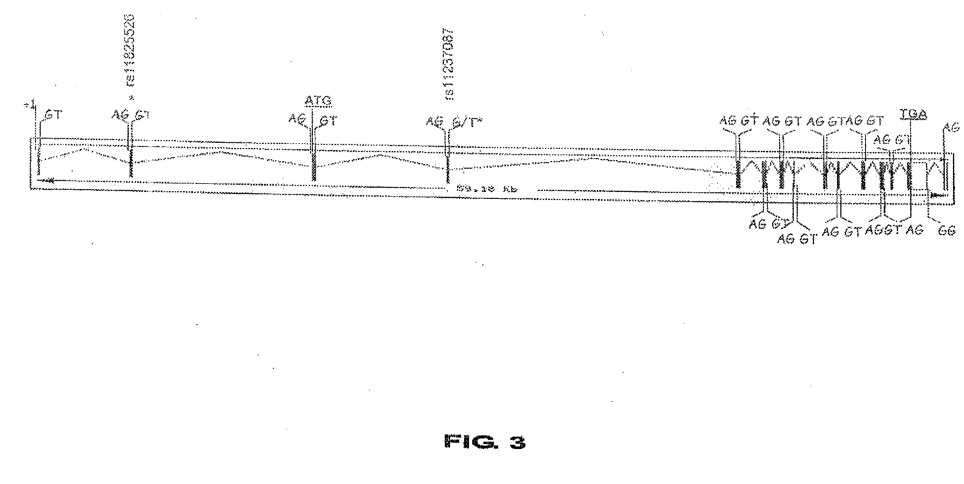

A second option was variant rs11237087. This polymorphism involves G>T substitution at the donor site of splicing of the fourth *CAPN5* exon. As shown in Figure 3, 14 of the 15 *CAPN5* exons have AG-GT consensus sites upstream and downstream from each intron. The target sequences of the splicing are strongly retained in *CAPN5.* Only intron 14 can be considered an exception to this rule. For this reason, this variant may generate an alternative splicing process in the CAPN5 mRNA, which would generate an aberrant protein.

**Table XVIII.** Characteristics of the rs1185523 and rs11237087 variants

| Gene | Transcript | SNP REF | Alleles | ESE | Value | Changes |
|---|---|---|---|---|---|---|
| ENSG00000149260 | ENST00000343564 | 11825526 | C/T | srp40 | 3.87 (C) | 2.34 (T)-Loss (-1.53) |
| | | 11825526 | C/T | srp40 | 3.54 (C) | 5.03 (T)-Maintains (1.49) |
| | | 11825526 | C/T | sf2 | 2.10 (C) | -0.85 (T) -Loss (-2.95) |
| | | 11825526 | C/T | sf2 | 3.52 (C) | 2.06 (T)-Maintains (-1.46) |
| | | | | Message | | |
| | | 11237087 | G/T | Intron 3-5' splicing point +2 | | |
| | | 11237087 | G/T | Intron 4-5' splicing point +2 | | |

The study described in Example 17 was carried out to confirm the possible contribution of these two variants to the development of the phenotypic charts referring to the relationship between CAPN5 and the alterations involving cardiovascular risk factors.

Example 17: Study of the possible involvement of variants rs11825526 and rs11237087 in the development of pathologies involving cardiovascular risk factors

A sub-cohort of 24 patients out of 606 initial patients used for the study of cardiovascular risk factors was selected, taking into consideration criteria that included either a severe manifestation of the symptoms that defined the charts, a cluster thereof or manifest cases of family clustering of the symptomatology. Their clinical and haplotype descriptions are included in Table XIX.

**Table XIX.** Clinical characteristics and haplotypes of the study population of variants rs11825526 and rs1123708

| Sample | Haplotype | Sex | BMI | WC | SP | DP | FG | G120 | FI | HOMA | CHOL | TRIG | HDL | LDL | Family background |
|--------|-----------|-----|------|-----|-------|------|-----|------|-------|------|------|------|-----|-------|-------------------|
| M2459 | GG | F | 29.25 | 103 | 120.0 | 70 | 83 | 131 | 13.53 | 2.75 | 238 | 102 | 62 | 155.6 | ND |
| M2365 | GG | M | 30.29 | 97 | 112.5 | 80 | 80 | 116 | 9.19 | 1.8 | 149 | 1.81 | 48 | 64.8 | ND |
| M2306 | GG | M | 25.39 | 85 | 110.0 | 65 | 70 | 66 | 6.68 | 1.14 | 165 | 48 | 45 | 110.4 | ND |
| CAF46 | HH | F | 21.94 | ND | ND | ND | ND | ND | ND | ND | ND | ND | ND | ND | DM and HC |
| CAF63 | HH | F | ND | ND | ND | ND | ND | ND | ND | ND | ND | ND | ND | ND | ND |
| CAF122 | HH | F | 26.14 | ND | ND | ND | ND | ND | ND | ND | 184 | 35 | | ND | ND |
| M2163 | HH | M | 27.11 | 93 | 135.0 | 90 | ND | ND | 13.18 | ND | ND | ND | ND | ND | ND |
| M2307 | DD | M | 18.81 | ND | 130.0 | 80 | 76 | 96 | 20.42 | 3.79 | 213 | 366 | 29 | 110.8 | ND |
| M2234 | DD | M | 30.12 | 94 | 127.5 | 92.5 | 86 | 65 | 16.96 | 3.57 | 298 | 178 | 39 | 223.4 | ND |
| CAF84 | DD | F | 19.14 | ND | ND | ND | ND | ND | ND | ND | 188 | 76 | ND | ND | DM and HBP |
| CAF91 | DD | F | 31.64 | ND | ND | ND | ND | ND | ND | ND | ND | ND | ND | ND | PCOS, DM, HBP and HC |
| CAF130 | BB | F | 29.59 | ND | ND | ND | ND | ND | ND | ND | ND | ND | ND | ND | DM, HBP and HC |
| M2521 | BB | F | 34.41 | 94 | 120.0 | 80 | ND | ND | 16.29 | ND | ND | ND | ND | ND | ND |
| M2241 | BB | M | 28.73 | 98 | 125.0 | 80 | 81 | 73 | 10.11 | 2 | 219 | 119 | 69 | 126.2 | ND |
| M2330 | BB | M | 33.55 | 94 | 147.5 | 95 | 120 | 187 | 30.09 | 8.83 | 307 | 193 | 61 | 207.4 | ND |
| M2392 | AA | M | 29.37 | 102 | 147.5 | 92.5 | 82 | 70 | 25.19 | 5.05 | 171 | 56 | 54 | 105.8 | ND |
| M2486 | AA | M | 26.85 | 97 | 150.0 | 90 | 92 | 102 | 16.02 | 3.6 | 242 | 160 | 66 | 144 | ND |
| CAF108 | EE | F | 19.49 | ND | ND | ND | ND | ND | ND | ND | ND | ND | ND | ND | DM and HBP |
| M2202 | CC | M | 32.21 | 110 | 130.0 | 90 | 112 | 119 | 9.67 | 2.65 | 184 | 95 | 65 | 100 | ND |
| M2412 | CC | F | 28.52 | 83 | 140.0 | 90 | 83 | 108 | 97.14 | 9.56 | 238 | 60 | 65 | 161 | ND |
| M2313 | AG | F | 23.55 | 81 | 135.0 | 80 | 75 | 126 | 9.44 | 1.73 | 192 | 47 | 93 | 89.6 | ND |
| M2354 | AE | M | 24.24 | 85 | 100.0 | 62.5 | 74 | 74 | 11.23 | 2.05 | 160 | 79 | 48 | 96.2 | ND |
| M2391 | DG | M | 19.77 | 79 | 105.0 | 62.5 | 61 | 66 | 10.15 | 1.51 | 86 | 78 | 57 | 23.4 | ND |

(continued)

| Sample | Haplotype | Sex | BMI | WC | SP | DP | FG | G120 | FI | HOMA | CHOL | TRIG | HDL | LDL | Family background |
|--------|-----------|-----|-------|-----|-------|-----|-----|------|-------|------|------|------|-----|-----|-------------------|
| M2595 | GF | M | 31.63 | ND | 180.0 | 90 | 112 | 284 | 24.20 | 6.92 | 257 | 115 | 50 | 184 | ND |

BMI Body mass index, WC: waist circumference (cm); SP: systolic pressure (mm Hg); DP: diastolic pressure (mm Hg); FG: fasting glucose (mg/dl), G120 (blood glucose 120 minutes after an oral overload (mg/dl); FI: fasting insulin (mg/dl); HOMA: homeostatic model; CHOL: total cholesterol (mg/dl); TRIG: triglyceride level in blood (mg/dl); HDL: HDL cholesterol (mg/dl); LDL: LDL cholesterol (mg/dl) ND: Not determined; DM: Diabetes Mellitus; HC: Hypercholesterolemia; HBP: High Blood Pressure; PCOS: Polycystic Ovary Syndrome

Using genomic DNA as a mold, the region comprising the ESE of exon 2 with the possible mutation was amplified and sequenced. None of the patients included in this study presented the described variation. In fact, a mixture of the DNA of 24 individuals belonging to the general population was carried out, and said sample was amplified, and after sequencing it, no heterozygosity could be observed in the studied locus. This data suggests that the variant rs11825526 could not be present in our population. On the other hand, the fact that this polymorphism has still not been validated by other independent studies suggests that it could be a highly infrequent variant or a sequencing error.

The hypothesis of the possible involvement of the rs1123708 variant was then started. To that end, the region corresponding to the intron-exon junction of interest in the patients belonging to the sub-cohort described previously was sequenced. A control mixture of 20 DNAs was also included in this study. The sequence analysis disclosed the absence of the G>T variant in the patients and in the control mixture.

The non-detection of the latter two variants analyzed in the population in which they were searched shows the difficulty in determining the gene variants that are really involved in the development of pathologies, disease and disorders.

**Description of the Figures**

Figure 1 shows the diagram of the *CAPN5* gene. The polymorphism sites and its clusters are indicated.
Figure 2 shows the *CAPN5* genotypes in the cases with PCOS against normal controls
X axis, genotypes, Y axis, % of genotypes with the PCOS cases (darker) and the controls (lighter)
Figure 3 shows the diagram of a primary transcript generated from the *CAPN5* gene showing the situation of the exons, the cutting and splicing sites of each exon and the location of the rs11825526 and rs11237087 mutations. ATG: initiation codon; TGA: stop codon. AG/GT Groups: cutting and splicing sites of each exon.

## Claims

1. An *in vitro* method in humans for the diagnosis or prognosis of the predisposition to suffer from polycystic ovary syndrome (PCOS), some of the pathologies associated thereto and/or at least one cardiovascular risk factor selected from: general obesity, abdominal obesity, hypertension, glucose intolerance, diabetes, hyperinsulinemia, general hypercholesterolemia, hypercholesterolemia with high LDL-cholesterol levels, low HDL-cholesterol levels and hypertriglyceridemia, or the grouping of some of these factors known as metabolic syndrome, **characterized in that** it detects a genotype or a haplotype of a polymorphism in the *CAPN5* gene or combinations thereof.

2.	An *in vitro* method according to claim 1, **characterized in that** it detects a genotype or a haplotype of a polymorphism selected from: Nt g.86 A>G, Nt g.344 G>A, Nt c.1320 C>T and Nt C.1469 G>A or combinations thereof.

3.	An *in vitro* method according to claim 2, **characterized in that** it preferably detects the genotype of the Nt g.86 A>G polymorphism for the diagnosis or prognosis: of PCOS, of at least one pathology associated thereto selected from amenorrhea and obesity; of the cardiovascular risk factor involved in hypercholesterolemia with high LDL-cholesterol levels.

4.	An in *vitro* method according to claim 2, **characterized in that** it preferably detects the genotype of the Nt g.344 G>A polymorphism for the diagnosis or prognosis of PCOS and/or of at least one pathology associated thereto selected from high blood pressure, gestational hypertension, type 2 diabetes mellitus, obesity and cancer, preferably nuclear cancer.

5.	An *in vitro* method according to claim 2, **characterized in that** it preferably detects the genotype of the Nt c.1320 C>T polymorphism for the diagnosis or prognosis of PCOS, of at least one pathology associated thereto which is type 2 diabetes mellitus, and/or of at least one cardiovascular risk factor selected from hypercholesterolemia with high LDL-cholesterol levels, diastolic hypertension, type 2 diabetes mellitus and obesity.

6.	An *in vitro* method according to claim 2, **characterized in that** it preferably detects a genotype of the Nt c.1469 G>A polymorphism for the diagnosis or prognosis: of PCOS; of at least one pathology associated thereto selected from amenorrhea and obesity; of the cardiovascular risk factor involved in hypercholesterolemia with high total cholesterol levels.

7.	An *in vitro* method according to claim 2, **characterized in that** it detects at least one common consensus haplotype -GC- for genotypes of the Nt g.86 A>G and Nt C.1469 G>A polymorphisms of the CAPN5 gene for the diagnosis and prognosis of PCOS.

8.	An *in vitro* method according to claim 2, **characterized in that** it detects at least the consensus haplotype -GCA for the diagnosis or prognosis of at least one cardiovascular risk factor selected from: general obesity, diastolic hypertension and glucose intolerance.

9.	An *in vitro* method according to claim 2, **characterized in that** it detects at least the consensus haplotype A-CG, for the diagnosis or prognosis of the metabolic syndrome.

10.	An *in vitro* method according to claim 2, **characterized in that** it detects at least the consensus haplotype A-CA, for the diagnosis or prognosis of at least one cardiovascular risk factor selected from: general obesity, abdominal obesity, diastolic hypertension, diabetes, glucose intolerance, and low HDL-cholesterol levels.

11.	An *in vitro* method according to claim 2, **characterized in that** it detects at least the consensus haplotype GG-G, for the diagnosis or prognosis of at least one cardiovascular risk factor selected from: hyperinsulinemia, general hypercholesterolemia and hypercholesterolemia with high LDL-cholesterol levels.

12.	An *in vitro* method according to claim 2, **characterized in that** it detects at least the consensus haplotype AGC-, for the diagnosis or prognosis of at least one cardiovascular risk factor selected from: general obesity, abdominal obesity, diastolic hypertension, diabetes, glucose intolerance, and low HDL-cholesterol levels.

13.	An *in vitro* method according to claim 2, **characterized in that** it detects at least the consensus haplotype AAC-, for the diagnosis or prognosis of at least one cardiovascular risk factor selected from obesity and metabolic syndrome.

14.	An *in vitro* method according to claim 2, **characterized in that** it detects at least the consensus haplotype GGC-, for the diagnosis or prognosis of at least one cardiovascular risk factor selected from: diastolic hypertension, diabetes, glucose intolerance, general hypercholesterolemia and hypercholesterolemia with high LDL-cholesterol levels.

15.	An in *vitro* method according to claims 2 to 14, **characterized in that** it detects two or more consensus haplotypes of the *CAPN5* gene selected from: -GCA, A-CG, A-CA, GG-G, AGC-, AAC- and GGC-for the diagnosis or prognosis of the metabolic syndrome.

16.	An in *vitro* method according to any of claims 2, 7, 8, 10 or 12, **characterized in that** it preferably detects at least the haplotype AGCA for the diagnosis or prognosis of PCOS and/or of at least one cardiovascular risk factor selected from: general obesity, abdominal obesity, diastolic hypertension, diabetes, glucose intolerance, general hypercholesterolemia, hypercholesterolemia with high LDL-cholesterol levels or low HDL-cholesterol levels.

17. An in *vitro* method according to any of claims 2 or 9, **characterized in that** it preferably detects at least the haplotype AGCG for the diagnosis or prognosis of the metabolic syndrome.

18. An in *vitro* method according to any of claims 2, 7, 11 or 14, **characterized in that** it preferably detects at least the haplotype GGCG for the diagnosis or prognosis of PCOS and/or of at least one cardiovascular risk factor selected from diastolic hypertension and hypercholesterolemia with high LDL-cholesterol levels.

19. An *in vitro* method according to any of claims 2 or 11, **characterized in that** it preferably detects at least the haplotype GGTG for the diagnosis or prognosis of at least one cardiovascular risk factor selected from: hyperinsulinemia, general hypercholesterolemia or hypercholesterolemia with high LDL-cholesterol levels.

20. An *in vitro* method according to any of claims 2 or 13, **characterized in that** it preferably detects at least the haplotype AACA for the diagnosis or prognosis of general obesity, low HDL-cholesterol levels or metabolic syndrome.

21. An *in vitro* method according to any of claims 2, 7 or 14, **characterized in that** it preferably detects at least the haplotype GGCA for the diagnosis or prognosis of PCOS or general hypercholesterolemia.

22. An *in vitro* method according to any of claims 2, 9 or 13, **characterized in that** it preferably detects at least the haplotype AACG for the diagnosis or prognosis of diastolic hypertension.

23. An *in vitro* method according to any of claims 2 to 6 and 8 to 21, **characterized in that** it detects combinations of two or more haplotypes of the *CAPN5* gene selected from: AGCA, AGCG, GGCG, GGTG, AACA, GGCA and AACG for the diagnosis or prognosis of the metabolic syndrome.

24. An in *vitro* method according to any of the previous claims, **characterized in that** the detection consists of a genotyping carried out by means of sequencing and/or fluorescence resonance energy transfer (FRET).

25. An assay device for the *in vitro* assay in humans for the diagnosis or prognosis of the predisposition to suffer from PCOS, any of the pathologies associated thereto and/or of at least one cardiovascular risk factor selected from general obesity, abdominal obesity, hypertension, glucose intolerance, diabetes, hyperinsulinemia, general hypercholesterolemia, hypercholesterolemia with high LDL-cholesterol levels, low HDL-cholesterol levels and hypertriglyceridemia, or the grouping of some of these factors known as metabolic syndrome, **characterized in that** it is based on the detection of at least one genotype or haplotype of a polymorphism in the *CAPN5* gene or combinations thereof using the method of claims 1 to 24.

26. An assay device according to claim 25, **characterized in that** it detects a genotype or a haplotype of a polymorphism selected from: Nt g.86 A>G, Nt g.344 G>A, Nt c.1320 C>T and Nt C.1469 G>A or combinations thereof.

27. An assay device according to claim 26, **characterized in that** it preferably detects the genotype of the Nt g.86 A>G polymorphism for the diagnosis or prognosis: of PCOS; of at least one pathology associated thereto selected from amenorrhea and obesity; of the cardiovascular risk factor involved in hypercholesterolemia with high LDL-cholesterol levels.

28. An assay device according to claim 26, **characterized in that** it preferably detects the genotype of the Nt g.344 G>A polymorphism, for the diagnosis or prognosis of PCOS and/or at least one pathology associated thereto selected from high blood pressure, gestational hypertension, type 2 diabetes mellitus, obesity and cancer, preferably nuclear cancer.

29. An assay device according to claim 26, **characterized in that** it preferably detects the genotype of the Nt c.1320 C>T polymorphism, for the diagnosis or prognosis of PCOS, of at least one pathology associated thereto which is type 2 diabetes mellitus, and/or of at least one cardiovascular risk factor selected from: hypercholesterolemia with high LDL-cholesterol levels, diastolic hypertension, type 2 diabetes mellitus and obesity.

30. An assay device according to claim 26, **characterized in that** it preferably detects a genotype of the Nt C.1469 G>A polymorphism, for the diagnosis or prognosis: of PCOS; of at least one pathology associated thereto selected from amenorrhea and obesity; of the cardiovascular risk factor involved in hypercholesterolemia with high total cholesterol levels.

31. An assay device according to claim 26, **characterized in that** it detects at least one common consensus haplotype -GC-for genotypes of the Nt g.86 A>G and Nt C.1469 G>A polymorphisms of the CAPN5 gene for the diagnosis and prognosis of PCOS.

32. An assay device according to claim 26, **characterized in that** it detects at least the consensus haplotype -GCA, for the diagnosis or prognosis of at least one cardiovascular risk factor selected from: general obesity, diastolic hypertension and glucose intolerance.

33. An assay device according to claim 26, **characterized in that** it detects at least the consensus haplotype A-CG, for the diagnosis or prognosis of the metabolic syndrome.

34. An assay device according to claim 26, **characterized in that** it detects at least the consensus haplotype A-CA, for the diagnosis or prognosis of at least one cardiovascular risk factor selected from: general obesity, abdominal obesity, diastolic hypertension, diabetes, glucose intolerance, and low HDL-cholesterol levels.

35. An assay device according to claim 26, **characterized in that** it detects at least the consensus haplotype GG-G, for the diagnosis or prognosis of at least one cardiovascular risk factor selected from: hyperinsulinemia, general hypercholesterolemia and hypercholesterolemia with high LDL-cholesterol levels.

36. An assay device according to claim 26, **characterized in that** it detects at least the consensus haplotype AGC-, for the diagnosis or prognosis of at least one cardiovascular risk factor selected from: general obesity, abdominal obesity, diastolic hypertension, diabetes, glucose intolerance, and low HDL-cholesterol levels.

37. An assay device according to claim 26, **characterized in that** it detects at least the consensus haplotype AAC-, for the diagnosis or prognosis of at least one cardiovascular risk factor selected from obesity and metabolic syndrome.

38. An assay device according to claim 26, **characterized in that** it detects at least the consensus haplotype GGC-, for the diagnosis or prognosis of at least one cardiovascular risk factor selected from: diastolic hypertension, diabetes, glucose intolerance, general hypercholesterolemia and hypercholesterolemia with high LDL-cholesterol levels.

39. An assay device according to any of claims 26 to 38, **characterized in that** it detects two or more consensus haplotypes of the *CAPN5* gene selected from: -GCA, A-CG, A-CA, GG-G, AGC-, AAC- and GGC-for the diagnosis or prognosis of the metabolic syndrome.

40. An assay device according to any of claims. 26, 31, 32, 34 or 36, **characterized in that** it preferably detects at least the haplotype AGCA for the diagnosis or prognosis of PCOS and/or of at least one cardiovascular risk factor selected from: general obesity, abdominal obesity, diastolic hypertension, diabetes, glucose intolerance, general hypercholesterolemia, hypercholesterolemia with high LDL-cholesterol levels or low HDL-cholesterol levels.

41. An assay device according to any of claims 26 or 33, **characterized in that** it preferably detects at least the haplotype AGCG for the diagnosis or prognosis of the metabolic syndrome.

42. An assay device according to any of claims 26, 31, 35 or 38, **characterized in that** it preferably detects at least the haplotype GGCG for the diagnosis or prognosis of PCOS and/or of at least one cardiovascular risk factor selected from diastolic hypertension and hypercholesterolemia with high LDL-cholesterol levels.

43. An assay device according to any of claims 26 or 35, **characterized in that** it preferably detects at least the haplotype GGTG for the diagnosis or prognosis of at least one cardiovascular risk factor selected from: hyperinsulinemia, general hypercholesterolemia or hypercholesterolemia with high LDL-cholesterol levels.

44. An assay device according to any of claims 26 or 37, **characterized in that** it preferably detects at least the haplotype AACA for the diagnosis or prognosis of general obesity, low HDL-cholesterol levels or metabolic syndrome.

45. An assay device according to any of claims 26, 31 or 38, **characterized in that** it preferably detects at least the haplotype GGCA for the diagnosis or prognosis of PCOS or general hypercholesterolemia.

46. An assay device according to any of claims 26, 33 or 37, **characterized in that** it preferably detects at least the haplotype AACG for the diagnosis or prognosis of diastolic hypertension.

47. A device according to any of claims 26 to 30 and 32 to 46, **characterized in that** it detects combinations of two or more haplotypes of the *CAPN5* gene selected from: AGCA, AGCG, GGCG, GGTG, AACA, GGCA and AACG for the diagnosis or prognosis of the metabolic syndrome.

48. An assay device according to any of claims 26 to 47, **characterized in that** the detection consists of a genotyping carried out by means of sequencing and/or fluorescence resonance energy transfer (FRET).

49. An assay device according to claim 48, **characterized by** genotyping a cluster of the Nt g.86 and Nt g.344 polymorphisms by PCR and automated sequencing using SEQ ID NO: 7 and SEQ ID NO: 8 as primers.

50. An assay device according to claim 48, **characterized by** genotyping a cluster of the Nt g.86 A>G and Nt g.344 G>A polymorphisms by PCR and automated sequencing, using SEQ ID NO: 7, SEQ ID NO: 9 and SEQ ID NO: 10 as primers for Nt g.86 A>G and SEQ ID NO: 11, SEQ ID NO: 12 and SEQ ID NO: 13 as primers for Nt g.344 G>A.

51. An assay device according to claim 48, **characterized by** genotyping a cluster of the Nt c.1320 C>T and Nt c.1469 G>A polymorphisms by fluorescence resonance energy transfer (FRET), using SEQ ID NO: 1 and SEQ ID NO: 2 as primers, and using SEQ ID NO: 3 or SEQ ID NO: 4 as probes for Nt c.1320 C>T or SEQ ID NO: 5 or SEQ ID NO: 6 as probes for Nt c.1469 G>A.

**FIG. 1**

EP 1 749 891 A1

FIG. 2

FIG. 3

# EP 1 749 891 A1

## INTERNATIONAL SEARCH REPORT

| International application No. |
|---|
| PCT/ ES 2005/070073 |

**A. CLASSIFICATION OF SUBJECT MATTER**

IPC$^7$ C12Q1/68,C12N9/50,A61K38/43

According to International Patent Classification (IPC) or to both national classification and IPC

**B. FIELDS SEARCHED**

Minimum documentation searched (classification system followed by classification symbols)

IPC$^7$ C12Q,C12N,A61K

Documentation searched other than minimum documentation to the extent that such documents are included in the fields searched

Electronic data base consulted during the international search (name of data base and, where practicable, search terms used)

CIBEPAT,EPODOC

**C. DOCUMENTS CONSIDERED TO BE RELEVANT**

| Category* | Citation of document, with indication, where appropriate, of the relevant passages | Relevant to claim No. |
|---|---|---|
| A | Gonzalez A. et al. "CAPN10 alleles are associated with polycystic ovary syndrome". J Clin Endocrinol Metab. Agosto 2002. Vol 87,n° 8, pages 3971-3976. | 1-24 |
| A | Legro RS. et al. "Molecular progress in infertility: polycistic ovary syndrome". Fertylity and Sterility. Septiembre 2002.Vol 78, n° 3, pages 569-576. | 1-24 |
| A | Meyer MF. et al. "Association of polycystic ovary syndrome with an interstitial deletion of the long arm of chromosome 11". Exp Clin Endocrinol Diabetes. 2000. Vol 108, pages 519-523. | 1-24 |

| [X] Further documents are listed in the continuation of Box C. | [X] See patent family annex. |
|---|---|

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 14 September 2005 (14.09.2005) | 21 September 2005 (21.09.2005) |

| Name and mailing address of the ISA/ | Authorized officer |
|---|---|
| SPTO | |
| Facsimile No. | Telephone No. |

Form PCT/ISA/210 (second sheet) (July 1992)

**INTERNATIONAL SEARCH REPORT**

| International application No. |
|---|
| PCT/ ES 2005/070073 |

C (Continuation).    DOCUMENTS CONSIDERED TO BE RELEVANT

| Category* | Citation of document, with indication, where appropriate, of the relevant passages | Relevant to claim No. |
|---|---|---|
| A | Huang Y. et al. "The calpain family and human disease".Trend in Molecular Medicine. Agosto 2001. Vol. 7, n° 8, pages 355-361. | 1-24 |
| A | Mugita N. et al. "Identification of a novel, tissue-specific calpain htra-3; a human homologue of the Caenorhabditis elegans sex determination gene". Biochem Biophys Res Commun. Octubre 1997. Vol. 239, n° 3, pages 845-50. | 1-24 |
| A | WO 03089623 A3 (NORTHWESTERN UNIVERSITY) 30.10.2003. | 1-24 |

Form PCT/ISA/210 (continuation of second sheet) (July 1992)

## INTERNATIONAL SEARCH REPORT

| International application No. |
|---|
| PCT/ ES 2005/070073 |

**Box I    Observations where certain claims were found unsearchable (Continuation of item 1 of first sheet)**

This international search report has not been established in respect of certain claims under Article 17(2)(a) for the following reasons:

1. ☐ Claims Nos.:
    because they relate to subject matter not required to be searched by this Authority, namely:

2. ☒ Claims Nos.:  1-24 (all in part), 25-31.
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

**Box II    Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**        ☐    The additional search fees were accompanied by the applicant's protest.
                             ☐    No protest accompanied the payment of additional search fees.

Form PCT/ISA/210 (continuation of first sheet (1)) (July 1992)

Continuation of Box I.2

Claims 1-24 (all in part), 25-30

1. Claims 1-24 relate to an undetermined number of possible methods for diagnosing multiple diseases involving the detection of a CAPN5 gene polymorphism genotype or haplotype (uncharacterised).

The claims as a whole lack clarity since the exact nature of the compound to be analysed in vitro has not been sufficiently characterised and the scope of the claims has not been sufficiently well defined as far as either the disease to be diagnosed or the parameter to be detected is concerned.

The phrase "characterised by the detection of a CAPN5 gene polymorphism genotype or haplotype or combinations thereof" is ambiguous and does not explicate the relationship between the genotypes and phenotypes claimed. It would be impossible for a person skilled in the art to determine clearly and unambiguously which of the genotypes claimed involve a predictive value related to the multiple diseases or risk factors. Table V indicates how the subject matter for which protection is sought can differ from the subject matter defined in the claims when the information in said table is used to interpret the claims. As a result, the claims as a whole lack clarity.

This lack of clarity is such that it has not been possible to carry out a meaningful search for all of the claims.

Consequently, the search has been directed to the part of the claims that appears to relate to the use of two polymorphisms localised to region 5', intron 1 of gene CAPN5: Nt g.86 A>G and Nt g.344 G>A (according to GenBank accession number AY547311) and to of two polymorphisms localised to the coding region of gene OMP, intron 3 of gene CAPN5: Nt C.1320C>T and Nt c. 1469 G>A (according to GenBank accession number U01212) for diagnostic or prognostic purposes.

2. Claims 25 to 30 characterise the test device in terms of the result to be achieved rather than the essential technical features of the device. The claims in this group lack the clarity required to carry out a meaningful search thereof.

Form PCT/ISA/210

| Patent document<br>cited in search report | Publication<br>date | Patent familiy<br>member(s) | Publication<br>date |
|---|---|---|---|
| WO 03089623 A2 | 30.10.2003 | AU 2003223726 A1 | 03.11.2003 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0023603 A

- WO 0245491 A

**Non-patent literature cited in the description**

- **BALKAU B ; CHARLES MA.** Comment on the provisional report from the WHO consultation, European Group for the Study of Insulin Resistance (EGIR. *Diabet Med.,* May 1999, vol. 16 (5), 442-443
- **BONFERRONI CE.** Teoría statistica delle classi e calcolo delle probabilitá. *Pubblicazioni del Istituto Superiore di Scienze Economiche e Commerciali di Firenze,* 1936, vol. 8, 3-62
- **BUCH B ; GALAN JJ ; LARA M ; RUIZ R ; SEGURA C ; REAL LM ; MARTINEZ-MOYA M ; RUIZ A.** Scanning of Y-chromosome azoospermia factors loci using real-time polymerase chain reaction and melting curve analysis. *Fertil Steril,* 2003, vol. 80 (4), 907-913
- **CAREY AH ; WATERWORTH D ; PATEL K ; WHITE D ; LITTLE J ; NOVELLI P ; FRANKS S ; WILLIAMSON R.** Polycystic ovaries and premature male pattern baldness are associated with one allele of the steroid metabolism gene CYP 17. *Hum Mol Genet,* 1994, vol. 3, 1873-1876
- **CARLSSON E ; FREDRIKSSON J ; GROOP L ; RIDDERSTRALE M.** Variation in the calpain-10 gene is associated with elevated triglyceride levels and reduced adipose tissue messenger ribonucleic acid expression in obese Swedish subjects. *J Clin Endocrinol Metab.,* 2004, vol. 89 (7), 3601-5
- **CARTEGNI L ; CHEW SL ; KRAINER AR.** Listening to silence and understanding nonsense: exonic mutations that affect splicing. *Nat Rev Genet,* 2002, vol. 3, 285-98
- **CARTEGNI L ; KRAINER AR.** Correction of disease-associated exon skipping by synthetic exon-specific activators. *Nat Struct Biol.,* 2003, vol. 10, 120-5
- **CHOBANIAN AV ; BAKRIS GL ; BLACK HR ; CUSHMAN WC ; GREEN LA ; IZZO JL JR ; JONES DW ; MATERSON BJ ; OPARIL S ; WRIGHT JT JR.** Joint National Committee on Prevention, Detection, Evaluation, and Treatment of High Blood Pressure. National Heart, Lung, and Blood Institute. *National High Blood Pressure Education Program*

- Coordinating Committee. Seventh report of the Joint National Cornmittee on Prevention, Detection, Evaluation, and Treatment of High Blood Pressure. *Hypertension,* 01 December 2003, vol. 42 (6), 1206-52
- **CONDE L ; VAQUERIZAS JM ; SANTOYO J ; AL.SHAHROUR F ; RUIZ-LLORENTE S ; ROBLEDO M ; DOPAZO J.** Pupa SNP Finder: a web tool for finding SNPs with putative effect at transcriptional level. *Nucleic Acids Res.,* 2004, vol. 32, W242-8
- **CROSIGNANI PG ; NICOLOSI AE.** Polycystic ovarian disease: heritability and heterogeneity. *Hum Reprod Update,* 2001, vol. 7, 3-7
- **DAIMON M ; OIZUMI T ; SAITOH T ; KARNEDA W ; YAMAGUCHI H ; OHNUMA H ; IGARASHI M ; MANABA H ; KATO T.** Calpain 10 gene polymorphisms are related, not to type 2 diabetes, but to increased serum cholesterol in Japanese. *Diabetes Res Clin Pract,* 2002, vol. 15 (56), 147-52
- **EAVES IA ; BENNETT ST ; FORSTER P ; FERBER KM ; EHRMANN D ; WILSON AJ ; BHATTACHARYYA S ; ZIEGLER AG ; BRINKMANN B ; TODD JA.** Transmission ratio distortion at the 1NSIGF2 VNTR. *Nat Genet,* 1999, vol. 22, 324-325
- **EHRRMANN DA ; SCHWARZ PE ; HARA M ; TANG X ; HORIKAWA Y ; IMPERIAL J ; BELL GI ; COX NJ.** Relationship of calpain-10 genotype co phenotypic features of polycystic ovary syndrome. *J Clin Endocrinol Metab,* 2002, vol. 87, 1669-1673
- **EHRRMANN DA ; TANG X ; YOSHIUCHI I ; COX NJ ; BELL GI.** Relationship of insulin receptor substrate-1 and -2 genotypes to phenotypic features of polycystic ovary syndrome. *J Clin Endocrinol Metab,* 2002, vol. 87, 4297-4300
- **ESCOBAR-MORREALE HF ; PERAL B ; VILLUENDAS G ; CALVO RM ; SANCHO J ; SAN MILLÁN JL.** Common single nucleotide polymorphisms in intron 3 of the calpain-10 gene influence hirsutism. *Fertil Steril,* 2002, vol. 77, 581-587

- **EVANS JC ; FRAYLING TM ; CASSELL PG ; SAKER PJ ; HITMAN GA ; WALKER M ; LEVY JC ; O'RAHILLY S ; RAO PV ; BENNETT AJ et al.** Studies of association between the gene for calpain-10 and type 2 diabetes mellitus in the United Kingdom. *Am J Hum Genet,* 2001, vol. 69, 544-552

- Expert Panel on Detection, Evaluation, and Treatment of High Blood Cholesterol in Adults. Executive Summary of The Third Report of The National Cholesterol Education Program (NCEP) Expert Panel on Detection, Evaluation, And Treatment of High Blood Cholesterol 1n Adults (Adult Treatment Panel 111). *JAMA,* 16 May 2001, vol. 285 (19), 248610 2497

- **FACKENTHAL JD ; CARTEGNI L ; KRAINER AR ; OLOPADE OI.** BRCA2 T2722R is a deleterious allele that causes exon skipping. *Am J Hum Genet.,* 2002, vol. 71, 625-31

- **GARANT MJ ; KAO WH ; BRANCATI F ; CORESH J ; RAMI TM ; HANIS CL ; BOERWINKLE E ; SHULDINER AR.** SNP43 of CAPNIO and the risk of type 2 Diabetes in African-Americans: the Atherosclerosis Risk in Communities Study. *Diabetes,* 2002, vol. 51, 231-237

- **GENUTH S ; ALBERTI KG ; BENNETT P ; BUSE J ; DERRONZO R ; KAHN R ; KITZMILLER J ; KNOWLER WC ; LEBOVITZ H ; LERNMARK A.** Expert Committee on the Diagnosis and Classification of Diabetes Mellitus. Follow-up report on the diagnosis of diabetes mellitus. *Diabetes Care.,* November 2003, vol. 26 (11), 3150-7

- **GHARANI N ; WATERWORTH DM ; BATTY S ; WHITE D ; GILLING-SMITH C ; CONWAY GS ; MCCARTHY M ; FRANKS S ; WILLIAMSON R.** Association of the steroid synthesis gene CYPllα with polycystic ovary syndrome and hyperandrogenism. *Hum Mol Genet,* 1997, vol. 6, 397-402

- **GONZÁLEZ A ; ABRIL E ; ROCA A ; ARAGON MJ ; FIGUEROA MJ ; VELARDE P ; ROYO JI ; REAL LM ; RUIZ A.** CAPN10 alleles are associated with polycystic ovary syndrome. *J Clin Endocrinol Metab,* 2002, vol. 87, 3971-3976

- **GONZÁLEZ A ; ABRIL E ; ROCA A ; ARAGON MJ ; FIGUEROA MJ ; VELARDE P ; RUIZ R ; FAYEZ O ; GALAN JJ ; HERREROS JA.** Specific CAPN10 gene haplotypes influence the clinical profile of polycystic ovary patients. *J Clin Endocrinol Metab,* 2003, vol. 588 (11), 5529-5536

- **GOVIND A ; OBHRAI MS ; CLAYTON RN.** polycystic ovaries are inherited as an autosomal dominant trait: analysis of 29 polycystic ovary syndrome and 10 control families. *J Clin Endocrinol Metab,* 1999, vol. 84, 38-43

- **HAFFNER SM ; KENNEDY E ; GONZALEZ C ; STERN MP ; MIETTINEN H.** A prospective analysis of the HOMA model. The Mexico City Diabetes Study. *Diabetes Care,* October 1996, vol. 19 (10), 113 8-41

- **HARDY GH.** Mendelian proportions in a mixed population. *Science,* 1908, vol. 28, 49-50

- **HONG J ; LI G ; LI C ; HUI R ; SUN S ; WANG J ; YE J ; CAI H.** Relationship between calpain-10 gene polymorphism, hypertension and plasma glucose. *Zhonghua Nei Ke Za Zhi,* 2002, vol. 41, 370-3

- **HORIKAWA Y ; ODA N ; COX NJ ; LI X ; ORHO-MELANDER M ; HARA M ; HINOKIO Y ; LINDNER TH ; MASHIMA H ; SCHWARZ PE.** Genetic variation in the gene encoding calpain-10 is associated with type 2 diabetes mellitus. *Nat Genet,* 2000, vol. 26, 163175

- **KRUSKAL WH ; WALLIS WA.** Use of ranks in one-criterion variance analysis. *J. Amer. Statist. Assoc.,* 1952, vol. 47, 583-621

- *J. AMER. STATIST. ASSOC.,* vol. 48, 907-911

- **LEGRO RS ; DRISCOLL D ; STRAUSS JF 3RD ; FOX J ; DUNAIF A.** Evidence for a genetic basis for hyperandrogenemia in polycystic ovary syndrome. *Proc Natl Acad Sci U S A,* 1998, vol. 95, 14956-14960

- **LEGRO RS ; STRAUSS JF.** Molecular progress in infertility: polycystic ovary syndrome. *Fertil Steril,* 2002, vol. 78, 569-576

- **LEWONTIN RC ; KOJIMA K.** The evolutionary dynamics of complex polymorphisms. *Evolution,* 1960, vol. 14, 450-472

- **MCKEIGUE P ; WILD S.** Association of insulin gene VNTR polymorphism with polycystic ovary syndrome. *Lancet,* 1997, vol. 349, 1771-1772

- **ONO Y ; SORIMACHI H ; SUZUKI K.** Structure and physiology of calpain, an enigmatic protease. *Biochem Biophys Res Commun,* 1998, vol. 245, 289-294

- **REAL LM ; GAYOSO AJ ; OLIVERA M ; CARUZ A ; RUIZ A ; GAYOSO F.** Detection of nucleotide c985 A-->G mutation of medium-chain acyl-CoA dehydrogenase gene by real-time PCR. *Clin Chem,* 2001, vol. 47, 958-959

- **RONAGHI M ; KARAMOHAMED S ; PETTERSSON B ; UHLEN M ; NYREN P.** Real-time DNA sequencing using detection of pyropnosphate release. *Anal. Biochem.,* 01 November 1996, vol. 242 (1), 84-89

- **SASIENI PD.** From genotypes to genes: doubling the sample size. *Biometrics,* 1997, vol. 53, 1253-1261

- **SHIMA Y ; NAKANISHI K ; ODAWARA M ; KOBAYASHI T ; OHTA H.** Association of the SNP-19 genotype 22 in the calpain-10 gene with elevated body mass index and hemoglobin Alc levels in Japanese. *Clin Chim Acta,* 2003, vol. 336, 89-96

- **SILANDER K. et al.** A large set of Finnish affected sibling pair families with type 2 diabetes suggests susceptibility loci on chromosomes 6, 11 and 14. *Diabetes,* 2004, vol. 53, 821829

- **SORIMACHI H ; ISHIURA S ; SUZUKI K.** Structure and physiological function of calpains. *Biochem J,* 1997, vol. 328, 721-732
- **SUZUKI H ; SAIDO TC ; HIRAI S.** Modulation of cellular signals by calpain. *Ann N Y Acad Sci,* 1992, vol. 674, 218-227
- **TALBOT JA ; BICKNELL EJ ; RAJKHOWA M ; KROOK A ; O'RAHILLY S ; CLAYTON RN.** Molecular scanning of the insulin receptor gene in women with polycystic ovarian syndrome. *J Clin Endocrinol Metab,* 1996, vol. 81, 1979-1983
- **TREGOUET DA ; BARBAUX S ; ESCOLANO S ; TAHRI N ; GOLMARD JL ; TIRET L ; CAMBIEN F.** Specific haplotypes of the P-selectin gene are associated with myocardial infarction. *Hum Mol Genet,* 2002, vol. 11, 2015-2023
- **TREGOUET DA ; BARBAUX S ; POIRIER O ; BLANKENBERG S ; BICKEL C ; ESCOLANO S ; RUPPC-ECHT HJ ; MEYER J ; CAMBIEN F ; TIRET L.** Gene Polymorphisms in Relation to Plasma SELPLG Levels and Coronary Artery Disease. *Ann. Hum Genet,* 2003, vol. 67, 504-511
- **TREGOUET DA ; ESCOLANO S ; TIRET L ; MALLET A ; GOLMARD JL.** A new algorithm for haplotype-based association analysis: the Stochastic-EM algorithm. *Ann Hum Genet.,* March 2004, vol. 68 (2), 165-77
- **URBANEK M ; LEGRO RS ; DRISCOLL DA ; AZZIZ R ; EHRMANN DA ; NORMAN RJ ; STRAUSS LII J,F ; SPIELMAN RS ; DUNAIF.** Thirty-seven candidate genes for polycystic ovary syndrome: strongest evidence for linkage is with follistatin. *Proc Natl Acad Sci U S A,* 1999, vol. 96, 8573-8578
- **WATERWORTH DM ; BENNETT ST ; GHARANI N ; MCCARTHY MI ; HAGUE S ; BATTY S ; CONWAY GS ; WHITE D ; TODD JA ; FRANKS S.** Linkage and association of insulin gene VNTR regulatory polymorphism with polycystic ovary syndrome. *Lancet,* 1997, vol. 349, 986-990
- **WEINBERG W.** Über den Nachweis der Verebung beim Menschen. *Jahresh. Verein f. Vaterl. Naturk in Wüttemberg,* 1908, vol. 64, 368-82
- **WEISS KM ; TERWILLIGER JD.** How many diseases does it take to map a gene with SNPs. *Nat Genet,* 2000, vol. 26, 151-157

**LIST OF SEQUENCES**

<110> NEOCODEX

<120> **METHOD AND DEVICE FOR THE IN VITRO DETECTION OF THE POLYCYSTIC OVARY SYNDROME (PCOS) AND OF PATHOLOGIES INVOLVING CARDIOVASCULAR RISK**

<130> PCT-194

<150> ES200401281

<151> 27.05.2004

<150> ES200402737

<151> 15.11.2004

<160> 13

<210> 1

<211> 18

<212> DNA

<213> Artificial sequence

<220>

<221> Primer

<223> OmpF

<400>
tggcctcttc tctccacg

<210> 2

<211> 18

<212> DNA

<213> Artificial sequence

<220>

<221> Primer

<223> OmpR

<400>
cctggccaag atccgcaa

<210> 3

```
<211> 19
<212> DNA
<213> Artificial sequence
<220>
<221> Probe
<223> 46 sensor
<220>
<223> fluorochrome Cyr in 5'
<220>
<223> phosphate in 3'


<400>
gcagatgcgg ctacgcgtg


<210> 4
<211> 21
<212> DNA
<213> Artificial sequence
<220>
<221> Probe
<223> 46 anchor
<220>
<223> fluorescein in 3'


<400>
cctggaccag ggcctgacca g


<210> 5
<211> 21
<212> DNA
<213> Artificial sequence
<220>
<221> Probe
<223> 49 sensor
<220>
<223> fluorochrome Rox in 5'
```

```
<220>
<223> phosphate in 3'

<400>
ggacaagccg ggcaaggtca c

<210> 6
<211> 25
<212> DNA
<213> Artificial sequence
<220>
<221> Probe
<223> 49 anchor
<220>
<223> fluorescein in 3'

<400>
cagttcgagc gctggaatgt cgtgc

<210> 7
<211> 21
<212> DNA
<213> Artificial sequence
<220>
<221> Primer
<223> capn5F
<220>
<223> marked with biotin

<400>
gatggtgact tagggagcag a

<210> 8
<211> 21
<212> DNA
<213> Artificial sequence
```

```
<220>
<221> Primer
<223> capn5R

<400>
gtcaactcca acttgcacat c

<210> 9
<211 > 20
<212> DNA
<213> Artificial sequence
<220>
<221> Primer
<223> CAPN5-76int

<400>
agccattgca gggaaggcca

<210> 10
<211> 14
<212> DNA
<213> Artificial sequence
<220>
<221> Primer
<223> CAPN5-76R

<400>
catttggtgg ctgc

<210> 11
<211> 18
<212> DNA
<213> Artificial sequence
<220>
<221> Primer
<223> CAPN5-140int
```

```
<220>
<223> marked with biotin

<400>
ccagtgccct cccacctt

<210> 12
<211> 21
<212> DNA
<213> Artificial sequence
<220>
<221> Primer
<223> CAPN5-R2

<400>
gtcaactcca acttgcacat c

<210> 13
<211> 20
<212> DNA
<213> Artificial sequence
<220>
<221> Primer
<223> CAPN5-Rpi

<400>
ggcacagcag ggtacaatgt
```